(19) Europäisches Patentamt / European Patent Office / Office européen des brevets

(11) **EP 4 238 991 A1**

(12) **EUROPEAN PATENT APPLICATION**
published in accordance with Art. 153(4) EPC

(43) Date of publication:
**06.09.2023 Bulletin 2023/36**

(21) Application number: **21894019.5**

(22) Date of filing: **19.11.2021**

(51) International Patent Classification (IPC):
$C07K\ 16/28$ (2006.01)   $C12N\ 15/13$ (2006.01)
$A61K\ 39/395$ (2006.01)   $A61P\ 35/00$ (2006.01)

(52) Cooperative Patent Classification (CPC):
**A61K 39/395; A61P 29/00; A61P 31/00;
A61P 35/00; A61P 37/02; C07K 16/00;
C07K 16/28; C12N 5/10; C12N 15/85**

(86) International application number:
**PCT/CN2021/131743**

(87) International publication number:
**WO 2022/105872 (27.05.2022 Gazette 2022/21)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.11.2020 CN 202011324100**

(71) Applicant: **Guangdong Fapon Biopharma Inc.
Dongguan, Guangdong 523000 (CN)**

(72) Inventors:
• **LU, Di
Dongguan, Guangdong 523000 (CN)**
• **HUO, Yongting
Dongguan, Guangdong 523000 (CN)**

• **LU, Lisheng
Dongguan, Guangdong 523000 (CN)**
• **HAN, Zhe
Dongguan, Guangdong 523000 (CN)**
• **HU, Peipei
Dongguan, Guangdong 523000 (CN)**
• **TU, Jingjing
Dongguan, Guangdong 523000 (CN)**
• **ZHANG, Chan
Dongguan, Guangdong 523000 (CN)**

(74) Representative: **De Clercq & Partners
Edgard Gevaertdreef 10a
9830 Sint-Martens-Latem (BE)**

Remarks:
The complete document including Reference Table(s) and the Sequence Listing(s) can be downloaded from the EPO website

(54) **ANTI-TIGIT ANTIBODY OR ANTIGEN-BINDING FRAGMENT THEREOF**

(57) Provided is an anti-TIGIT antibody or an antigen-binding fragment thereof. The antibody or the antigen-binding fragment thereof has a higher affinity for TIGIT, has functional characteristics in a plurality of aspects, and may be used alone or in combination with other reagents for treating cancers, and diseases related to immunity and inflammation.

EP 4 238 991 A1

Fig. 10B

Binding epitope competition experiment of Tigit mouse monoclonal antibody and R0300 and CHO-hTIGIT cells

Processed by Luminess, 75001 PARIS (FR)

## Description

### Technical Field

The present disclosure relates to the field of biomedicine, and specifically to an anti-TIGIT antibody or an antigen-binding fragment thereof.

### Background

A T cell Ig and ITIM domain (TIGIT, also called WUCAM, Vstm3 or VSIG9) is an inhibitory receptor shared by T cells containing Immunoglobulin Tail Tyrosine (ITT) motif structural domains and Immunoreceptor Tyrosine-based Inhibitory Motif (ITIM) structural domains, and NK cells, belongs to a type I transmembrane protein, and includes an IgV extracellular domain and an ITT-like phosphorylation fragment.

The gene was discovered by a research group of Genentech in 2008. The research group found the gene by searching genes in a genome that met specific conditions (① expressing on immune cells; ② belonging to the type I transmembrane protein; ③ extracellularly carrying immunoglobulin structural domains; and ④ intracellularly carrying immunoregulation structural domains). By means of sequence alignment, it is discovered that this protein belongs to a large ligand Poliovirus Receptor (PVR) protein family, whose members included activated receptors CD226 and CD96, which competed with TIGIT for ligands, and ligand PVR thereof, and the like. In 2012, the crystal structure of the TIGIT molecule was analyzed by X-ray diffraction; it was found that the TIGIT expressed on immune cells first forms a cis-homodimer on the same cell, and then the dimer binds a PVR molecule by means of the TIGIT molecule on each side; and it was also found that the pre-formed homodimer is necessary for TIGIT-PVR interaction because the TIGIT-PVR interaction is disrupted if the amino acids at a TIGIT-TIGIT binding interface are mutated in advance.

The main ligands of the TIGIT are CD155 (Necl-5, PVR) and CD112(PVRL2, Nectin-2). Among the above, the binding affinity with CD155 is the highest (3.15nM, Kd). Two TIGIT molecules and two CD155 molecules form a tetramer "lock-key structure" (Try113 of the TIGIT and AX6G of the CD155, and Phe128 of the CD155 and AX6G of the TIGIT respectively form the "key-lock" structure). These two ligands are also the ligands of CD226(DNAM-1); and the CD226 competes with the TIGIT, so as to stimulate the activity of the T cells. The interaction between the ligands and the TIGIT defeats the CD226, resulting in suppression of immune activity and upregulation of CD155 and CD122 by tumor cells to evade immune-mediated destruction. Therefore, antagonistic antibodies specific for the TIGIT may inhibit the suppression of CD155 and CD112-induced T-cell responses and enhance antitumor immunity.

### Summary

The present disclosure is intended to obtain an antibody specifically recognizing TIGIT or an antigen-binding fragment thereof. Heavy chain CDR1, CDR2 and CDR3 are selected from at least one of combinations of SEQ ID NOs:1-3, SEQ ID NOs:4-6, SEQ ID NOs:7-9, SEQ ID NOs:10-12, SEQ ID NOs:13-15, SEQ ID NOs:16-18, or SEQ ID NOs:19-21;
light chain CDR1, CDR2 and CDR3 are selected from at least one of SEQ ID NOs:22-24, SEQ ID NOs:25-27, SEQ ID NOs:28-30, SEQ ID NOs:31-33, SEQ ID NOs:34-36, SEQ ID NOs:37-39, or SEQ ID NOs:40-42.

The present disclosure further provides a nucleic acid, a vector and a cell, which are related to the antibody or the antigen-binding fragment thereof.

The present disclosure further relates to a method for producing the antibody or the antigen-binding fragment thereof as described above. The method includes:

The cell as described above is cultured in a culture medium, and

An antibody produced from the culture medium or the cultured cell is recovered.

Another aspect of the present disclosure further relates to an application of the antibody or the antigen-binding fragment thereof as described above in manufacture of a medicament for treating or preventing infectious diseases, immune diseases or tumors.

The present disclosure further provides a kit. The kit includes at least one of the following components:

i) the antibody or the antigen-binding fragment thereof as described above, and an optional container used for holding the antibody or the antigen-binding fragment thereof; or

ii) the pharmaceutical composition as described above, and an optional container used for holding the pharmaceutical composition.

The antibody has a higher affinity for the TIGIT, has functional characteristics in a plurality of aspects, and may be used alone or in combination with other reagents for treating cancers, and diseases related to immunity and inflammation.

## Brief Description of the Drawings

In order to more clearly illustrate the specific implementations of the present disclosure or the technical solutions in the related art, the drawings used in the description of the specific implementations or the related art will be briefly described below. It is apparent that the drawings in the following descriptions are some implementations of the present disclosure. Other drawings can be obtained from those skilled in the art according to these drawings without any creative work.

Fig. 1A, Fig. 1B and Fig. 1C show binding affinity of Tigit-5, 3Tigit-12, 3Tigit-16, 3Tigit-24, 3Tigit-29, 14Tigit-1-1 and 14Tigit-3-2 to CHO-hTIGIT verified by means of Fluorescence-Activated Cell Sorting (FACS) according to an embodiment of the present disclosure.

Fig. 2A, Fig. 2B and Fig. 2C show binding affinity of Tigit-5, 3Tigit-16, 3Tigit-24, 3Tigit-29, 14Tigit-1-1 and 14Tigit-3-2 to Peripheral Blood Mononuclear Cells (PBMC) in healthy people verified by means of FACS according to an embodiment of the present disclosure.

Fig. 3A and Fig. 3B show a blocking effect of Tigit-5, 3Tigit-16, 3Tigit-24, 3Tigit-29 and 14Tigit-3-2 on the binding of CHO-hTIGIT and hPVR-hFc verified by means of FACS according to an embodiment of the present disclosure.

Fig. 4A and Fig. 4B show a blocking effect of Tigit-5, 3Tigit-16, 3Tigit-24, 3Tigit-29, 14Tigit-1-1 and 14Tigit-3-2 on the binding of CHO-hPVR and hTIGIT-hFc verified by means of FACS according to an embodiment of the present disclosure.

Fig. 5A and Fig. 5B show a blocking effect of Tigit-5, 3Tigit-16, 3Tigit-24, 3Tigit-29, 14Tigit-1-1 and 14Tigit-3-2 on the binding of a tumor cell and a TIGIT verified by means of FACS according to an embodiment of the present disclosure.

Fig. 6A and Fig. 6B show a blocking effect of Tigit-5, 3Tigit-16, 3Tigit-24, 3Tigit-29, 14Tigit-1-1 and 14Tigit-3-2 on the binding of CHO-CD112 and a TIGIT verified by means of FACS according to an embodiment of the present disclosure.

Fig. 7A and Fig. 7B show the binding ability of Tigit-5, 3Tigit-16, 3Tigit-29 and 14Tigit-1-1 to cynoTIGIT verified by means of FACS according to an embodiment of the present disclosure.

Fig. 8A and Fig. 8B show verification of an activation effect of Tigit-5, 3Tigit-12, 3Tigit-16 and 3Tigit-24 on a killing function of NK92-hTIGIT according to an embodiment of the present disclosure.

Fig. 9 shows verification of the capabilities of Tigit-5 and 3Tigit-16 to activate NK92-hTIGIT to kill U2-OS according to an embodiment of the present disclosure.

Fig. 10A shows verification of whether different antibodies are the same as R0223 epitope according to an embodiment of the present disclosure.

Fig. 10B shows verification of whether different antibodies are the same as R0300 epitope according to an embodiment of the present disclosure.

## Detailed Description of the Embodiments

Reference to implementations of the present disclosure, one or more examples of which are described below, will now be provided in detail. Each example is provided as illustration and non-limitation to the present disclosure. In fact, it is apparent to those skilled in the art that various modifications and variations may be made in the present disclosure without departing from the scope or spirit of the present disclosure. For example, features illustrated or described as a part of one implementation may be used in another implementation, to produce a still further implementation.

Therefore, it is intended that the present disclosure covers such modifications and variations falling within the scopes of the appended claims and equivalents thereof. Other objects, features and aspects of the present disclosure are disclosed in or are apparent from the following detailed descriptions. It should be understood by those of ordinary skill in the art that this discussion is the description of exemplary implementations only and is not intended to limit the broader aspects of the present disclosure.

The present disclosure relates to an antibody, which is an antibody or an antigen-binding fragment thereof binding a human TIGIT. Heavy chain CDR1, CDR2 and CDR3 are selected from at least one of combinations of SEQ ID NOs:1-3, SEQ ID NOs:4-6, SEQ ID NOs:7-9, SEQ ID NOs:10-12, SEQ ID NOs:13-15, SEQ ID NOs:16-18, or SEQ ID NOs:19-21;

light chain CDR1, CDR2 and CDR3 are selected from at least one of SEQ ID NOs:22-24, SEQ ID NOs:25-27, SEQ ID NOs:28-30, SEQ ID NOs:31-33, SEQ ID NOs:34-36, SEQ ID NOs:37-39, or SEQ ID NOs:40-42.

In some implementations, the antibody or the antigen-binding fragment thereof has any one of the following CDR sequence combinations:

| | H-CDR1 | H-CDR2 | H-CDR3 | L-CDR1 | L-CDR2 | L-CDR3 |
|---|---|---|---|---|---|---|
| Tigit-5 | SEQ ID NO:1 | SEQ ID NO:2 | SEQ ID NO:3 | SEQ ID NO: 22 | SEQ ID NO: 23 | SEQ ID NO: 24 |
| 3Tigit-16 | SEQ ID NO:4 | SEQ ID NO:5 | SEQ ID NO:6 | SEQ ID NO:25 | SEQ ID NO:26 | SEQ ID NO:27 |
| 3Tigit-24 | SEQ ID NO:7 | SEQ ID NO:8 | SEQ ID NO:9 | SEQ ID NO: 28 | SEQ ID NO: 29 | SEQ ID NO: 30 |
| 3Tigit-29 | SEQ ID NO: 10 | SEQ ID NO: 11 | SEQ ID NO: 12 | SEQ ID NO: 31 | SEQ ID NO: 32 | SEQ ID NO: 33 |
| 14Tigit-1-1 | SEQ ID NO: 13 | SEQ ID NO: 14 | SEQ ID NO: 15 | SEQ ID NO: 34 | SEQ ID NO: 35 | SEQ ID NO: 36 |
| 14Tigit-3-2 | SEQ ID NO: 16 | SEQ ID NO: 17 | SEQ ID NO: 18 | SEQ ID NO: 37 | SEQ ID NO: 38 | SEQ ID NO: 39 |
| 3Tigit-12 | SEQ ID NO: 19 | SEQ ID NO: 20 | SEQ ID NO: 21 | SEQ ID NO: 40 | SEQ ID NO: 41 | SEQ ID NO: 42 |

An important advantage of the antibody or the antigen-binding fragment thereof lies in that the antibody or the antigen-binding fragment thereof has higher affinity for the TIGIT.

An important advantage of the antibody or the antigen-binding fragment thereof lies in that the antibody or the antigen-binding fragment thereof has the activity of blocking the binding of CD112 and the TIGIT.

An important advantage of the antibody or the antigen-binding fragment thereof lies in that the antibody or the antigen-binding fragment thereof has the activity of blocking the binding of PVR and the TIGIT.

An important advantage of the antibody or the antigen-binding fragment thereof lies in that the antibody or the antigen-binding fragment thereof has the activity of activating the killing capability of NK92-TIGIT.

An important advantage of the antibody or the antigen-binding fragment thereof lies in that the binding epitope of the antibody or the antigen-binding fragment thereof to the TIGIT is new.

Due to the above characteristics, the antibody or the antigen-binding fragment thereof may be preferably used as an antibody medicine for use.

According to the anti-TIGIT antibody or the antigen-binding fragment thereof of the present disclosure, an inhibition signal of cancer cells to immune cells can be counteracted by means of signal transmission of TIGIT/CD155 interaction, and reactivation of an immune response is induced to effectively attack the cancer cells, such that an anti-cancer effect is provided. Finally, the anti-TIGIT antibody or the antigen-binding fragment thereof may be used for immunotherapy against cancer targeting the TIGIT (a tumor immunosuppressive agent). In particular, according to the anti-TIGIT antibody or the antigen-binding fragment thereof of the present disclosure, the expression or activity of the TIGIT in a subject with cancer is reduced or inhibited, and a persistent anticancer response of a T cell or an NK cell is induced, such that the effect of treating the cancer is provided.

In the present disclosure, the technical term of "antibody or antigen-binding fragment thereof" is a protein binding a specific antigen, which refers broadly to all proteins and protein fragments including a Complementarity Determinant Region (CDR). "Antibody" particularly refers to a full-length antibody. The term "full-length antibody" includes a polyclonal antibody and a monoclonal antibody; and the term "antigen-binding fragment" is a substance containing a part or all of antibody CDRs, and the fragment lacks at least some of amino acids present in a full-length chain but can still be capable of specifically binding to an antigen. Such fragments are biologically-active because the fragments bind to target antigens and may competitively bind to given epitope with other antigen-binding molecules (including a complete antibody). In some implementations, the antigen-binding fragment has the effect of specifically recognizing and binding the TIGIT. In some implementations, the antigen-binding fragment is a fragment that has the function of blocking the binding CD112 and the TIGIT, and/or blocking the binding PVR and the TIGIT, and/or activating the killing capability of NK92-TIGIT. In one aspect, such fragments may contain a single heavy chain and a single light chain, or portions thereof. The fragment may be generated by a recombinant nucleic acid technology, or may be generated by enzymatic cleavage or chemical cleavage of the antigen-binding molecules (including the complete antibody).

The term "Complementarity Determinant Region" or "CDR" refers to a hypervariable region of heavy chains and light chains of immunoglobulins. There are three heavy chain CDRs and three light chain CDRs. Herein, depending on the situation, the term "CDR" and "CDRs" are used for referring to regions containing one or more or even all of major amino acid residues that contribute to the binding affinity of an antibody or an antigen-binding fragment thereof to the antigen or epitope by which the antibody or the antigen-binding fragment thereof recognizes. In another specific implementation, the CDR refers to a hypervariable region of the heavy chain and the light chain of the immunoglobulins that is defined by IMGT.

In the present disclosure, a CDR of a heavy chain is represented by HCDR, and a CDR of a light chain is represented by LCDR. A CDR labeling method commonly used in the art includes: a Kabat numbering scheme, Chothia and Lesk numbering schemes, and a new standardized numbering system introduced by Lefranc et al. in 1997 for all protein sequences of an immunoglobulin superfamily. Kabat et al. are the first to propose a standardized numbering scheme for an immunoglobulin variable region. In their compilation Sequences of Proteins of Immunological Interest, amino acid sequences of a light chain ($\lambda$, $\kappa$) variable region and an antibody heavy chain, as well as a variable region ($\alpha$, $\beta$, $\gamma$ and $\delta$) of a T-cell receptor, are aligned and numbered. Over the past decades, the accumulation of sequences led to the creation of a KABATMAN database, and the Kabat numbering scheme is often considered as a standard, which is widely used for numbering antibody residues. In the present disclosure, a Kabat annotation standard is used to label the CDR, but other methods of labeling the CDR also fall within the scope of protection of the present disclosure.

The terms "specific recognition", "selective binding", "selectively bound" and "specifically bound" or similar expressions refer to the binding of an antibody or an antigen-binding fragment thereof to predetermined epitope on an antigen. Generally, the antibody or the antigen-binding fragment thereof binds with an affinity ($K_D$) of about less than $10^{-6}$M, for example, about less than $10^{-7}$M, $10^{-8}$M, $10^{-9}$M, or $10^{-10}$ or less.

In the present disclosure, subjects that are recognized by the provided antibody or the antigen-binding fragment thereof may be TIGITs from a plurality of generic sources, such as humans, mice, and monkeys (such as cynomolgus monkeys).

The variants of the antibody or the antigen-binding fragment thereof also fall within the scope of the present disclosure, for example, sequences respectively having at least 70%, 80%, 85%, 90%, 91%, 92%, 93%, 94%, 95%, 96%, 97%, 98%, 99% or greater than 99% identity to each CDR or FR, or variable regions VL and/or VH, or amino acid sequences or nucleotide sequences of the full length of the antibody described in the present disclosure. In some cases, the variants of the antibody or the antigen-binding fragment thereof at least include the above 6 CDRs. In some cases, the variants of the antibody or the antigen-binding fragment thereof at least include one heavy chain and one light chain, and in other cases, a variant form contains two identical light chains and two identical heavy chains (or sub-portions thereof). In some cases, the variants of the antibody or the antigen-binding fragment thereof are obtained by means of conservative modification or conservative substitution or substitution on the sequences of the antibody or the antigen-binding fragment thereof provided in the present disclosure. "Conservative modification" or "conservative substitution or substitution" refers to the substitution of amino acids in a protein by other amino acids with similar features (such as charge, side chain size, hydrophobicity/hydrophilicity, main chain conformation and rigidity), causing frequent changes without altering the biological activity of the protein. It is known to those skilled in the art that, in general, single amino acid substitution in a non-essential region of a polypeptide does not substantially alter the biological activity (see, for example, Watson et al. (1987)Molecular Biology of the Gene, The Benjamin/Cummings Pub.Co. Page 224, (4th edition)). In addition, substitution of structurally or functionally similar amino acids is unlikely to break the biological activity. In some cases, the variants retain the capability of blocking the binding of the TIGIT and ligand CD112 or PVR thereof. A person skilled in the pertinent art will be able to determine suitable variants of the antigen-binding molecules as set forth herein using technologies well known. In some implementation solutions, a person skilled in the pertinent art may identify suitable regions of a molecule that may be altered by targeting regions believed to be unimportant for activity without disrupting the activity. For nucleotide and amino acid sequences, the term "identity" indicates the degree of identity between two nucleic acid sequences or two amino acid sequences during optimal comparison and contrast with appropriate insertion or deletion.

In some implementations, heavy chain FR1, FR2, FR3 and FR4 of the antibody or the antigen-binding fragment thereof are selected from at least one of combinations of SEQ ID NOs:43-46, SEQ ID NOs:47-50, SEQ ID NOs:51-54, SEQ ID NOs:55-58, SEQ ID NOs:59-62, SEQ ID NOs:63-66, or SEQ ID NOs:67-70;

light chain FR1, FR2, FR3 and FR4 of the antibody or the antigen-binding fragment thereof are selected from at least one of SEQ ID NOs:71-74, SEQ ID NOs:75-78, SEQ ID NOs:79-82, SEQ ID NOs:83-86, SEQ ID NOs:87-90, SEQ ID NOs:91-94, or SEQ ID NOs:95-98.

Optionally, the antibody or the antigen-binding fragment further includes at least one of the following framework region sequence combinations:

|  | H-FR1 | H-FR2 | H-FR3 | H-FR4 | L-FR1 | L-FR2 | L-FR3 | L-FR4 |
|---|---|---|---|---|---|---|---|---|
| Tigit-5 | SEQ ID NO:43 | SEQ ID NO:44 | SEQ ID NO:45 | SEQ ID NO:46 | SEQ ID NO:71 | SEQ ID NO:72 | SEQ ID NO: 73 | SEQ ID NO:74 |
| 3Tigit-16 | SEQ ID NO:47 | SEQ ID NO:48 | SEQ ID NO:49 | SEQ ID NO:50 | SEQ ID NO:75 | SEQ ID NO:76 | SEQ ID NO:77 | SEQ ID NO:78 |
| 3Tigit-24 | SEQ ID NO:51 | SEQ ID NO:52 | SEQ ID NO:53 | SEQ ID NO:54 | SEQ ID NO:79 | SEQ ID NO:80 | SEQ ID NO:81 | SEQ ID NO:82 |
| 3Tigit-29 | SEQ ID NO:55 | SEQ ID NO:56 | SEQ ID NO:57 | SEQ ID NO:58 | SEQ ID NO:83 | SEQ ID NO:84 | SEQ ID NO:85 | SEQ ID NO:86 |
| 14Tigit-1-1 | SEQ ID NO:59 | SEQ ID NO:60 | SEQ ID NO:61 | SEQ ID NO:62 | SEQ ID NO:87 | SEQ ID NO:88 | SEQ ID NO:89 | SEQ ID NO:90 |
| 14Tigit-3-2 | SEQ ID NO:63 | SEQ ID NO:64 | SEQ ID NO:65 | SEQ ID NO:66 | SEQ ID NO:91 | SEQ ID NO:92 | SEQ ID NO:93 | SEQ ID NO:94 |
| 3Tigit-12 | SEQ ID NO:67 | SEQ ID NO:68 | SEQ ID NO:69 | SEQ ID NO:70 | SEQ ID NO:95 | SEQ ID NO:96 | SEQ ID NO:97 | SEQ ID NO:98 |

The variable regions of the heavy chains and the light chains are obtained by means of combination in a manner of FR1-CDR1-FR2-CDR2-FR3-CDR3-FR4.

In a preferred implementation, the variable regions of the heavy chain and the light chain combine CDRs and FRs one-to-one according to the content in Tigit-5, 3Tigit-16, 3Tigit-24, 3Tigit-29, 14Tigit-1-1, 14Tigit-3-2, and 3Tigit-12 rows in the above table.

In some implementations, the antigen-binding fragment is any one of Fab, F(ab')2, Fd, Fv, scFv, a bispecific antibody, or a minimal recognition unit of the antibody, and preferably, one of the F(ab')$_2$, the Fab, the scFv, or the bispecific antibody.

In some implementations, the antibody has a constant region, a heavy chain constant region sequence is selected from a constant region sequence of any one of IgG1, IgG2, IgG3, IgG4, IgA, IgM, IgE, or IgD, and a light chain constant region is a κ or a λ chain.

In some implementations, the species source of the constant region is cattle, horses, pigs, sheep, goats, rats, mice, dogs, cats, rabbits, camels, donkeys, deer, minks, chickens, ducks, geese, or humans.

The present disclosure further relates to a nucleic acid, which encodes the antibody or the antigen-binding fragment thereof as described above.

The nucleic acid is usually RNA or DNA, and the nucleic acid molecule may be single-stranded or double-stranded, but preferably double-stranded DNA. The nucleic acid is "effectively linked" when being placed in a functional relationship with another nucleic acid sequence. For example, if a promoter or an enhancer affects the transcription of an encoding sequence, the promoter or the enhancer is effectively linked to the encoding sequence. The nucleic acid preferably uses a DNA nucleic acid when being linked into a vector.

In addition, in view of the antibody being a membrane protein, the nucleic acid usually carries a signal peptide sequence.

The present disclosure further relates to a vector, which includes the nucleic acid as described above.

The term "vector" refers to a nucleic acid delivery vehicle into which polynucleotide may be inserted. When the vector enables the expression of a protein encoded by the inserted polynucleotide, the vector is called an expression vector. The vector may be introduced into a host cell by means of transformation, transduction or transfection, so as to obtain the expression of a genetic material elements carried by the vector in the host cell. The vector is well known to those skilled in the art, and includes, but is not limited to: a plasmid; a phagemid; a cosmid; an artificial chromosome, such as a Yeast Artificial Chromosome (YAC), a Bacteriophage Artificial Chromosome (BAC) or a P1-derived Artificial chromosome (PAC); and a phage, such as a λ phage or an M13 phage and animal viruses. The animal viruses that may be used as the vectors include, but are not limited to, retroviruses (including lentiviruses), adenoviruses, adeno-associated viruses, herpesviruses (such as herpes simplex virus), poxviruses, baculoviruses, papillomaviruses, and papovavirus (such as SV40). In some implementations, the vector described in the present disclosure includes regulatory elements commonly used in genetic engineering, such as enhancers, promoters, Internal Ribosome Entry Sites (IRES), and other expression control elements (such as transcription termination signals, or polyadenylation signals, and poly-U sequences).

The present disclosure further provides a cell, which includes the nucleic acid as described above or the vector as described above.

The expressions "cell", "cell line" and "cell culture" are used interchangeably herein, and all such names include progeny. Therefore, the terms "transformant" and "transformed cell" include both the primary recipient cell and the cultures derived from the primary recipient cell, regardless of the number of transfers. It should also be understood that, due to intentional or unintentional mutations, all offspring are unlikely to be precisely the same in terms of DNA content, including mutant progeny with the same function or biological activity as those screened in the initially transformed cells. In the case of meaning different names, it is clearly visible by the context.

The host cell or cell line suitable for expressing the antigen-binding protein of the present disclosure includes mammalian cells, such as NS0, Sp2/0, CHO, COS, HEK, fibroblasts, and myeloma cells. Human cells may be used, such that molecules are allowed to be modified with human glycosylation patterns. Alternatively, other eukaryotic cell lines may be used. Selection of appropriate mammalian host cells, and methods for transformation, culture, amplification, screening and product generation and purification are known in the art.

It can be proved that, bacterial cells may be used as the host cells, which are suitable for expressing recombinant Fab or other implementation solutions of the present disclosure. However, since proteins expressed in the bacterial cells tend to be in unfolded forms or incorrectly folded forms or non-glycosylated forms, any recombinant Fab produced in the bacterial cells must be screened to retain an antigen-binding capability. If the molecules expressed by the bacterial cells are produced in a properly folded form, the bacterial cells are desired hosts; or in a replaceable implementation solution, the molecules may be expressed in the bacterial cells, and then re-folding is subsequently performed. For example, various *Escherichia coli* strains for expression are host cells well-known in the field of biotechnology. Various strains such as *bacillus subtilis, streptomyces* and other *bacillus sp* may also be used in the method.

If required, yeast cell strains known to those skilled in the art, as well as insect cells, such as drosophila and lepidoptera and a viral expression system, may also be used as host cells.

Another aspect of the present disclosure further relates to a method for producing the antibody or the antigen-binding fragment thereof as described above. The method includes the following operations:

the cell as described above is cultured in an appropriate culture condition, and

an antibody or an antigen-binding fragment thereof produced from a culture medium or the cultured cell is recovered.

Common methods for producing the antibody or the antigen-binding fragment thereof include in vitro cultivation of the cells, such as hybridomas, and collection of the antibody or the antigen-binding fragment thereof from the supernatant.

Alternatively, the cell is transplanted into an immunodeficient animal (such as naked mice), and then the antibody or the antigen-binding fragment thereof is collected and purified from positions such as animal serum.

The present disclosure further provides a pharmaceutical composition. The pharmaceutical composition includes the antibody or the antigen-binding fragment thereof as described above, and one or more of pharmacologically acceptable excipients, diluents or carriers.

The terms "pharmacologically acceptable excipients, diluents or carriers" refer to excipients, diluents or carriers that are pharmacologically and/or physiologically compatible with a subject and active ingredient.

According to another aspect of the present disclosure, the present disclosure further relates to an application of the antibody or the antigen-binding fragment thereof as described above in manufacture of a medicament for treating or preventing infectious diseases, immune diseases or tumors.

The present disclosure further relates to a kit. The kit includes at least one of the following components:

i) the antibody or the antigen-binding fragment thereof as described above, and an optional container used for holding the antibody or the antigen-binding fragment thereof; or

ii) the pharmaceutical composition as described above, and an optional container used for holding the pharmaceutical composition.

In addition, the present disclosure further relates to a method for treating the disease of a subject. The method includes administering the effective amount of the antibody or the antigen-binding fragment thereof as described above to a subject, and the antibody or the antigen-binding fragment thereof is optionally combined with another therapeutic agent or a treatment process.

The disease is selected from infectious diseases, immune diseases or tumors.

In some implementations, the subject is an animal.

In some implementations, the subject is a mammal.

In some implementations, the subject is a primate.

In some implementations, the subject is human.

The term "optionally" is for descriptive purposes only and should not be construed as indicating or implying relative importance. Therefore, features delimited with "optionally" may expressly or implicitly include the feature.

The implementations of the present disclosure will be described in detail below with reference to embodiments.

**Embodiment 1 Production and Preparation of Anti-human TIGIT antibody**

An anti-human TIGIT monoclonal antibody was produced on the basis of a conventional hybridoma technique, and specific processes include the following.

1. Antigen preparation:

A soluble human TIGIT fusion protein internally prepared or commercially purchased (AcroBiosystems, Catalog#TIT-H5253) was used as an antigen for immunization.

The internally prepared antigen was selected from a human TIGIT extracellular segment; a mouse mIgG2a-Fc fragment was fused on the C-terminal of the segment; transient expression was performed in an HEK293 cell with a standard recombinant protein expression technology; secretory expression supernatant was collected from the cell; and a target protein was obtained by means of purification with a protein A affinity chromatography column, and was split and stored, after sterilization and filtration, at -80°C for later use.

2. Mouse immunization:

In order to produce the anti-human TIGIT monoclonal antibody, 6-10-week Balb/c female mice were immunized with the human TIGIT-mFc protein as described previously. The human TIGIT-mFc protein was injected intraperitoneally or subcutaneously into the immunized mice, with a dosage being $25\mu g/100\mu L$, one injection was given per week, and 6 injections were totally given for immunization; or the dosage was $50\mu g/100\mu L$, one injection was given every two weeks, and 5 injections were totally given for immunization; the specific number of injections for immunization was related to an actual immune effect of each mouse; and generally, subsequent steps were performed on the mice with desirable immune effects in advance, and the mice with poor immune effects were continued to be immunized. The immune effect was determined by monitoring serum titer in mice; and generally, after 3 injections for immunization, the binding capability (that is, the titer) of mouse serum to the antigen was detected by means of an ELISA method. The mice with the highest titer were selected for booster immunization; 50 μg of the antigen was used and injected through the spleen; and fusion was ready after 3 days of booster immunization.

3. Hybridoma fusion:

A conventional technology was used to take the spleens of the mice, mouse spleen cells were obtained by means of separation; and mouse tumor cells SP2/0 and immunized spleen cells were mixed in a ratio of 1:10 cell numbers, were transferred into a 50 ml centrifuge tube, and were washed once with an RPMI1640 basic culture medium. Supernatant was discarded; the cells were well mixed; and 1ml of 50%PEG1500 was slowly added for fusion. After 1 min of fusion, 15 ml of the RPMI1640 basic culture medium was added to terminate cell fusion. Centrifugation was performed for 5 min at 1000rpm, and the supernatant was discarded. 50 ml of an RPMI1640 screening culture medium was used to perform gentle suspending, and was evenly divided in 96-well plates, 10 pieces in total; and culture was performed in a 37°C and 5%$CO_2$ cell incubator in a standing manner in $50\mu l$/well. Culture was performed to Day 6, an HT culture medium (an RPMI1640 complete culture medium containing HT) was replaced twice.

Fusion detection: a 0.05M carbonate buffer solution was used to dilute a human TIGIT recombinant protein (internally prepared) carrying a human Fc tag to a final concentration of $2\mu g/ml$, and was added into a 96-well ELISA detection plate in 100 μL/well; and incubation was performed for 2h at 37°C or coating was performed overnight at 2-4°C. Supernatant was discarded; after washing was performed for 5 times with a plate washing solution ($1\times$PBS), a blocking solution ($1\times$PBS+1%BSA) was added in $200\mu l$/well; and blocking was performed for 1h at 37°C. At Day 7 after recombinant fusion, cell supernatant was added in 100 μl/well, and incubation was performed for 30 min at 37°C in a standing manner. Washing was performed for 3 times with the 1×PBS. HRP-labeled goat anti-mouse IgG (sigma A0168-1ML) was added in 100 μl/well; incubation was performed for 30 min at 37°C in a standing manner; and after washing was performed for 3 times with the 1×PBS, a color reaction was performed, and then data was read at OD450/OD630nm on a microplate reader. Fusions with OD450nm-OD630nm≥1.0 were selected; and cloning was performed with a limiting dilution method for at least 2 times.

Positive subclone detection: a human TIGIT recombinant protein (internally prepared) carrying a human Fc Tag was diluted with a 0.05M carbonate buffer solution to a final concentration of 2μg/ml, and was added into a 96-well ELISA detection plate in 100 μL/well; and incubated for 2h at 37°C or coating was performed overnight at 2-4°C. Supernatant was discarded; after washing was performed for 5 times with the 1×PBS, the blocking solution (1×PBS+1%BSA) was added in 200μl/well; and blocking was performed for 1h at 37°C. Subclone supernatant was added in 100μl/well, and incubation was performed for 30 min at 37°C in a standing manner. Washing was performed for 3 times with the 1×PBS. HRP-labeled goat anti-mouse IgG (sigma A0168-1ML) was added in 100 μl/well; incubation was performed for 30 min at 37°C in a standing manner; and after washing was performed for 3 times with the 1×PBS, a color reaction was performed, and then data was read at OD450/OD630nm on a microplate reader. The obtained positive subclone was cultured in vitro for conservation and expression.

4. Expression and purification of mouse monoclonal antibody:

After a clone producing the anti-human TIGIT antibody grew until a convergence rate reached 80%, cells were blown down; after centrifugation was performed for 5 min at 1000rpm, the cells were resuspended with 30 ml of an SFM complete culture medium containing 5% FBS, and the culture medium was transferred to a 150ml SF shake flask; and culture was performed for 2-3 days at 37°C, 8%$CO_2$ and 120rpm. When cell density reached 5×10E5/ml, centrifugation was performed for 5 min at 1000rpm; the cells were resuspended with a 50 ml serum free SFM basic culture medium, and the culture medium was transferred to a 250ml SF shake flask; culture was performed for 4-5 days at 37°C, 8%$CO_2$ and 120rpm; and centrifugation was performed for 20 min at 9000rpm, and supernatant is collected for purification. Pro A affinity chromatography was used for purification. A process includes the following. An AKTA avant 150 chromatography device was used; at least a 5CV equilibration buffer (10mM PBS) was used to equilibrate a chromatography column (for example, MabSelectSuRe LX, GE); and a sample was loaded to the chromatography column, so as to cause a target protein to be adsorbed on the chromatography column and cause other impurities to penetrate and to be separated. After sample loading, the at least 5CV equilibration buffer (10mM PBS) was used to wash the chromatography column again; then an elution buffer (20mM NaAc, pH=3.4) was used to elute the target protein; a neutralizing buffer (1M Tris, pH8.0) was added in a collection tube in advance; and the adding volume of the neutralizing buffer was determined according to estimated content of an elution sample, and 10% elution volume was added generally.

Concentration measurement was performed on the sample with Biotek-Epoch-Take-3; an A280 method was used to detect the concentration of the antibody, that is, with extinction coefficient E.C.=1.37, optical path=0.05mm (Take-3 plates having slight differences in the optical path of different wells, which would be automatically corrected); a light absorption value of the sample was detected by means of a device; and the concentration of an antibody to be measured was calculated according to Lambert-Beer law. If the concentration of the sample was too low, ultrafiltration concentration needed to be performed; the sample was concentrated to > 0.5 mg/ml with an ultrafiltration concentration tube (Amicon® Ultra-15Centrifugal Filter Devices, 30kD) according to a general operation method provided in the specification; and a concentrated end sample was collected, and sterilization and filtration were performed with a 0.22um sterile needle filter (Cobetter, PES, 0.22um, and diameter being 13 mm), and then split charging and freeze storage were performed for later use.

5. Identification of mouse monoclonal antibody subtypes:

A 0.05M pH9.5 carbonate buffer solution was used to dilute a recombinant human TIGIT recombinant protein (internally prepared) carrying a human Fc Tag to a final concentration of 2μg/mL, and was added into a 96-well ELISA detection plate in 100 μL/well; and incubation was performed for 2h at 37°C or coating was performed overnight at 2-4°C. Supernatant was discarded; after washing was performed for 5 times with the 1×PBS, the blocking solution (1×PBS+1%BSA) was added in 200μl/well; and blocking was performed for 1h at 37°C. The antibody was diluted to 1μg/ml with 1×PBS containing 1%BSA; the obtained anti-human TIGIT mouse monoclonal antibody was added in 100 μl/well; and incubation was performed for 30 min at 37 °C. Washing was performed for 3 times with the 1×PBS. HRP-labeled goat anti-mouse IgG(sigma A0168-1ML), HRP-labeled goat anti-mouse IgG2a (thermo fisher M32207), HRP-labeled goat anti-mouse IgG1 (thermo fisher PA1-74421), HRP-labeled goat anti-mouse IgG2b(thermo fisher M32407), HRP-labeled goat anti-mouse IgG3 (thermo fisher M32607) and HRP-labeled goat anti-mouse IgM (thermo fisher 31440) were added in 100 μl/well; incubation was performed for 30 min at 37°C in a standing manner; and after washing was performed for 3 times with the 1×PBS, the color reaction was performed, and then the data was read at OD450/OD630nm on the microplate reader.

**Embodiment 2 Binding, Blocking and Screening of Anti-TIGIT Antibody.**

1. Detection of the mouse monoclonal antibody binding to CHO-hTIGIT:

The CHO-engineered cells CHO-hTIGIT cells recombinantly expressing full-length human TIGIT to be detected were counted, centrifuged at 300 g for 5 min, resuspended in an FCM buffer and cell density was adjusted to 4E+06 cell/mL for later use. A sample to be detected (including a positive control antibody and a mouse monoclonal antibody) was diluted with the FCM buffer to 20μg/mL, and then the FCM buffer was used to perform 3x serial dilutions of 12 concentration points; and the isotype control antibody was diluted with the FCM buffer to 20μg/mL. According to an experimental design, in each well, 50μL of the antibody and 50μL of the cell (the amount of each cell added into a 96-well V-shaped plate being 2E+05 cells/well) were respectively added in the 96-well V-shaped plate; and incubation in dark was performed on ice for 30 min. Centrifugation (300g/5min) was performed; supernatant was discarded; 200μL of the FCM buffer was added for washing once; centrifugation (300g/5min) was performed; and the supernatant was discarded. Secondary antibodies (PE Goat anti-mouse IgG, 1:500 dilution) were added to the 96-well V-shaped plate (in 100μL/well); and incubation was performed in dark on ice for 30 min. Centrifugation (300g/5min) was performed; supernatant was discarded; 200μL of the FCM buffer was added once for washing once; centrifugation (300g/5min) was performed; and the supernatant was discarded. 100μL of the 1×PBS was added for resuspending, and on-machine detection was performed.

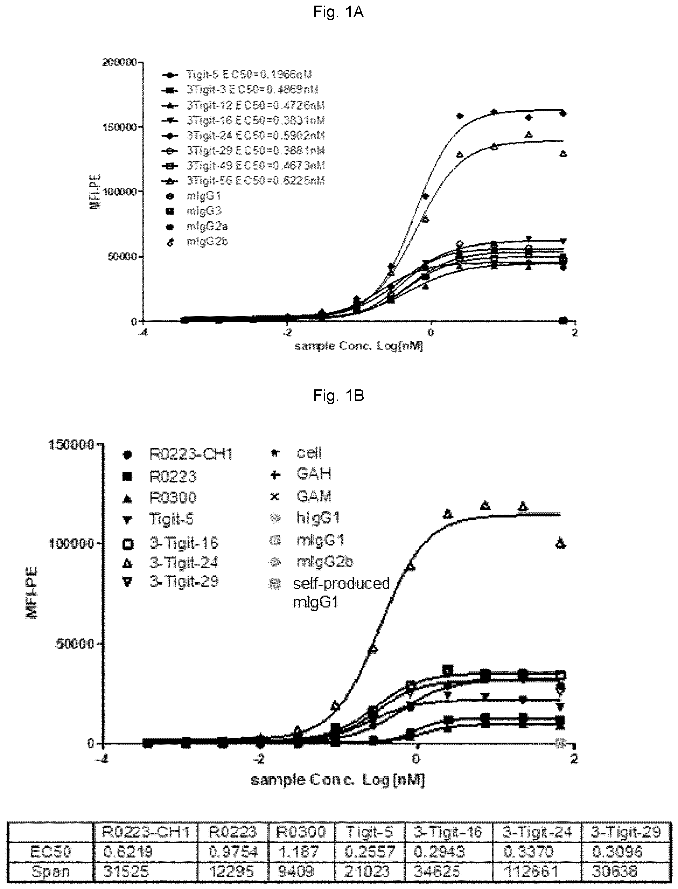

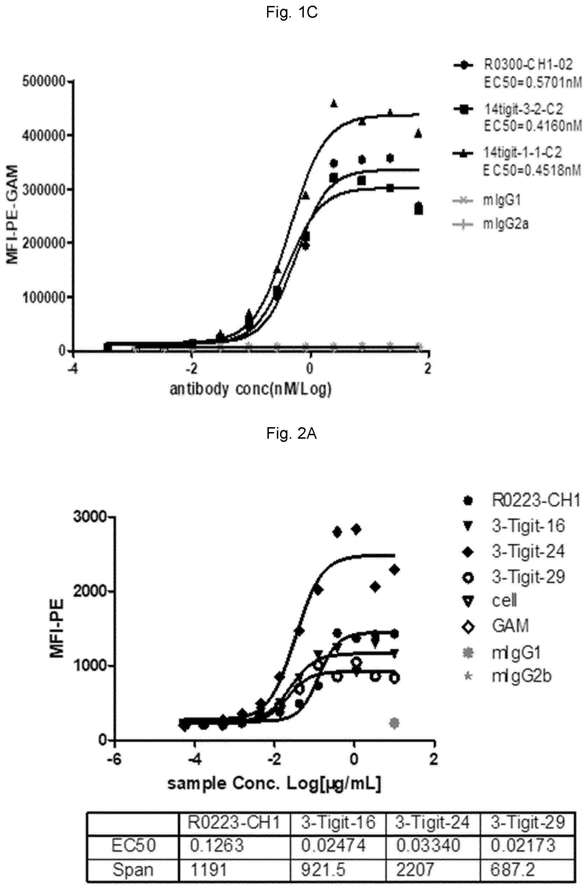

By means of result analysis, R0223 was derived from 22G2IgG1.1f in the patent application WO 2016106302(A1), and R0223-CH1 was the Fc region of the R0223 replaced with the Fc region of mIgG1. R0300 was derived from tiragolumab in the patent application WO 2017053748(A2), and R0300 was the Fc region replaced with the Fc region of mIgG1. The R0223 and the R0300 were used as screening control antibodies. From results of Fig. 1A, Fig. 1B and Fig. 1C, it could be seen that, the binding affinity of Tigit-5, 3Tigit-16, 3Tigit-24, 3Tigit-29, 14Tigit-1-1 and 14Tigit-3-2 to CHO-hTIGIT is superior to that of the control antibodies.

2. Detection of the mouse monoclonal antibody binding to PBMC in healthy people:

In order to verify that the prepared anti-human TIGIT antibody may effectively bind to naturally expressed TIGIT, the following experiments were specifically performed. PBMC cells in healthy people to be detected were counted, centrifuged at 300 g for 5 min, resuspended with an FCM buffer; and cell density was adjusted to 4E+06 cell/mL for later use. Samples to be detected (including a positive control antibody and a mouse monoclonal antibody) were diluted with the FCM buffer to 20μg/mL, and then the FCM buffer was used to perform 3x serial dilutions of 12 concentration points; and the isotype control antibody was diluted with the FCM buffer to 20μg/mL. According to an experimental design, in each well, 50μL of the antibody and 50μL of the cell (the volume of each cell added into a 96-well V-shaped plate being 2E+05 cell/well) were respectively added in the 96-well V-shaped plate; and incubation was performed in dark on ice for 30 min. Centrifugation (300g/5min) was performed; supernatant was discarded; 200μL of the FCM buffer was added for washing once; centrifugation (300g/5min) was performed; and the supernatant was discarded. Secondary antibodies (PE Goat anti-mouse IgG, 1:500 dilution) were added to the 96-well V-shaped plate (in 100μL/well); and incubation was performed in dark on ice for 30 min. Centrifugation (300g/5min) was performed; supernatant was discarded; 200μL of the FCM buffer was added for washing once; centrifugation (300g/5min) was performed; and the supernatant was discarded. 100μL of the 1×PBS was added for resuspending, and on-machine detection was performed.

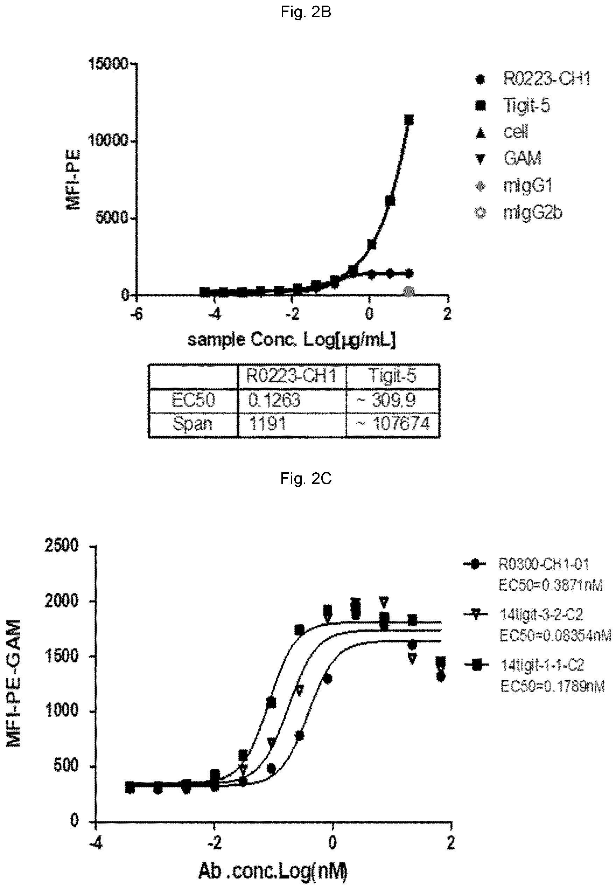

By means of result analysis, from results of Fig. 2A, Fig. 2B and Fig. 2C, it could be seen that, the binding affinity of Tigit-5, 3Tigit-16, 3Tigit-24, 3Tigit-29, 14Tigit-1-1 and 14Tigit-3-2 to the PBMC in healthy people is superior to that of the control antibodies.

3. Detection the mouse monoclonal antibody blocking the binding of CHO-hTIGIT and hPVR-hFc:

In order to verify that the anti-human TIGIT antibody may block the binding of CHO-hTIGIT and hPVR-hFc, the following experiments were specifically performed. The CHO-engineered cells CHO-hTIGIT cells recombinantly expressing full-length human TIGIT were counted, and centrifugated at 300 g for 5 min, resuspended with an FCM buffer, and cell density was adjusted to 4E+06 cell/mL for later use. Samples to be detected (including a positive control antibody and a mouse monoclonal antibody) were diluted with the FCM buffer to 20μg/mL, and then the FCM buffer was used to perform 3x serial dilutions of 12 concentration points; a ligand protein hPVR-hFc was diluted with the FCM buffer to 20μg/mL; and an isotype control antibody was diluted with the FCM buffer to 20μg/mL. According to an experimental design, in each well, 50μL of the antibody and 50μL of the cell (the volume of each cell added into a 96-well V-shaped plate being 2E+05 cell/well) were respectively added in the 96-well V-shaped plate; and incubation was performed in dark on ice for 30 min. The ligand protein was added to the 96-well V-shaped plate (in 50μL/well); and incubation was performed in dark on ice for 30 min. Centrifugation (300g/5min) was performed; supernatant was discarded; 200μL of the FCM buffer was added for washing once; centrifugation (300g/5min) was performed; and the supernatant was discarded. Secondary antibodies (PE anti-human IgG Fc, 1:500 dilution) were added to the 96-well V-shaped plate (in 100μL/well); and incubation was performed in dark on ice for 30 min. Centrifugation (300g/5min) was performed; supernatant was discarded; 200μL of the FCM buffer was added for washing once; centrifugation (300g/5min) was performed; and the supernatant was discarded. 100μL of the 1×PBS was added for resuspending, and on-machine detection was performed.

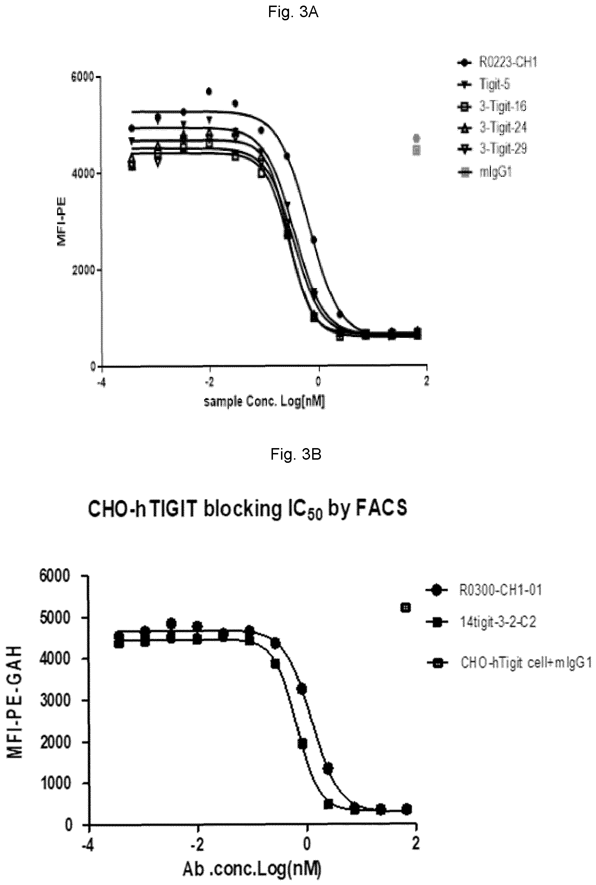

By means of result analysis, from results of Fig. 3A and Fig. 3B, it could be seen that, Tigit-5, 3Tigit-16, 3Tigit-24, 3Tigit-29 and 14Tigit-3-2 can well block the binding of CHO-hTIGIT and hPVR-hFc, and the blocking effect is better than that of the control antibodies.

4. Detection the mouse monoclonal antibody blocking the binding of CHO-hPVR and hTIGIT-hFc:

In order to verify that the anti-human TIGIT antibody may block the binding of CHO-hPVR and hTIGIT-hFc, the following experiments were specifically performed. Samples to be detected (including a positive control antibody and a mouse monoclonal antibody) were diluted with the FCM buffer to 40μg/mL, and then the FCM buffer was used to perform 3x serial dilutions of 12 concentration points; an antigen protein hTIGIT-hFc was diluted with the FCM buffer to 0.6μg/mL; and an isotype control antibody was diluted with the FCM buffer to 40μg/mL. The antibodies to be detected and the antigen protein hTIGIT-hFc were added to the 96-well V-shaped plate (the antibodies and the antigens were added in 50μL/well); and incubation was performed in dark on ice for 30 min. The CHO-engineered cells CHO-hPVR cells recombinantly expressing full-length human PVR were counted centrifugated at 300 g for 5 min, resuspended with an FCM buffer, the cells were added to the 96-well V-shaped plate; the volume of each cell added into a 96-well V-shaped plate was 2E+05 cell/well (being added in 50μL/well); and incubation was performed in dark on ice for 30 min. Centrifugation (300g/5min) was performed; supernatant was discarded; 200μL of the FCM buffer was added for washing once; centrifugation (300g/5min) was performed; and the supernatant was discarded. Secondary antibodies (PE anti-human IgG Fc, 1:500 dilution) were added to the 96-well V-shaped plate (in 100μL/well); and incubation was performed in dark on ice for 30 min. Centrifugation (300g/5min) was performed; supernatant was discarded; 200μL of the FCM buffer was added for washing once; centrifugation (300g/5min) was performed; and the supernatant was discarded. 100μL of the 1×PBS was added for resuspending, and on-machine detection was performed.

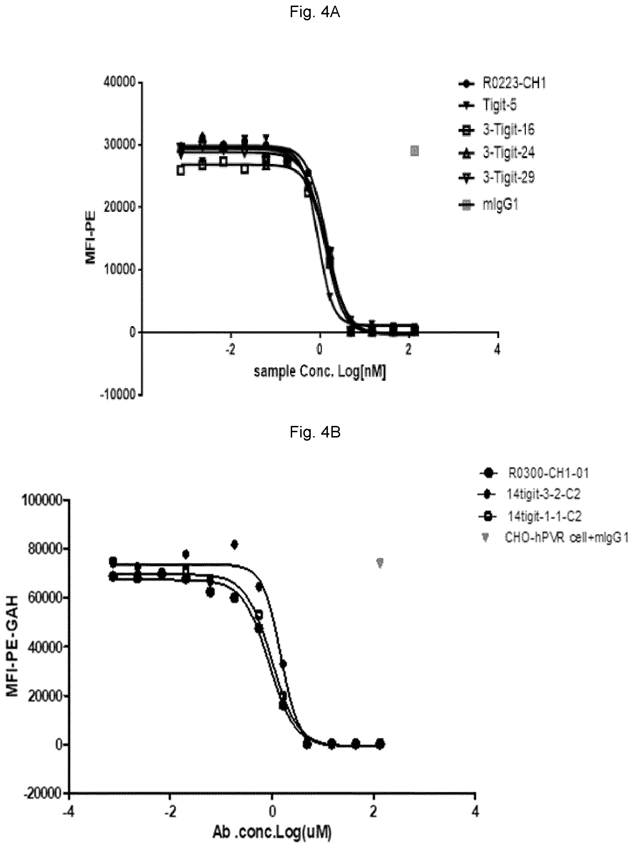

By means of result analysis, from results of Fig. 4A and Fig. 4B, it could be seen that, Tigit-5, 3Tigit-16, 3Tigit-24, 3Tigit-29, 14Tigit-1-1 and 14Tigit-3-2 can well block the binding of CHO-hPVR and hTIGIT-hFc, and the blocking effect is better than that of the control antibodies.

5. Detection the mouse monoclonal antibody blocking natural tumor cells from binding hTIGIT-hFc

In order to verify that the anti-human TIGIT antibody blocks the binding of hPVR expressed by natural tumor cells and hTIGIT-hFc, the following experiments were specifically performed. U2-OS cells to be detected (human osteosarcoma cells, naturally and highly expressing PVR and CD112 by means of detection) were counted; centrifugated at 300 g for 5 min; resuspended with an FCM buffer; and cell density was adjusted to 4E+06 cell/mL for later use. Samples to be detected (including a positive control antibody and a mouse monoclonal antibody) were diluted with the FCM buffer to 20μg/mL, and then the FCM buffer was used to perform 3x serial dilutions of 12 concentration points; an antigen protein hTIGIT-hFc was diluted with the FCM buffer to 120μg/mL; and an isotype control antibody was diluted with the FCM buffer to 20μg/mL. According to an experimental design, in each well, 50μL of the antigen protein and 50μL of the cell (the volume of each cell added into a 96-well V-shaped plate being 2E+05 cell/well) were respectively added in the 96-well V-shaped plate; and incubation was performed in dark on ice for 30 min. Centrifugation (300g/5min) was performed; supernatant was discarded; 200μL of the FCM buffer was added for washing once; centrifugation (300g/5min) was performed; and the supernatant was discarded. The antibody was added to the 96-well V-shaped plate (in 100μL/well); and incubation was performed in dark on ice for 30 min. Centrifugation (300g/5min) was performed; supernatant was discarded; 200μL of the FCM buffer was added for washing once; centrifugation (300g/5min) was performed; and the supernatant was discarded. Secondary antibodies (APC anti-human IgG Fc, 1:500 dilution) were added to the 96-well V-shaped plate (in 100μL/well); and incubation was performed in dark on ice for 30 min. Centrifugation (300g/5min) was performed; supernatant was discarded; 200μL of the FCM buffer was added for washing once; centrifugation (300g/5min) was performed; and the supernatant was discarded. 100μL of the 1×PBS was added for resuspending, and on-machine detection was performed.

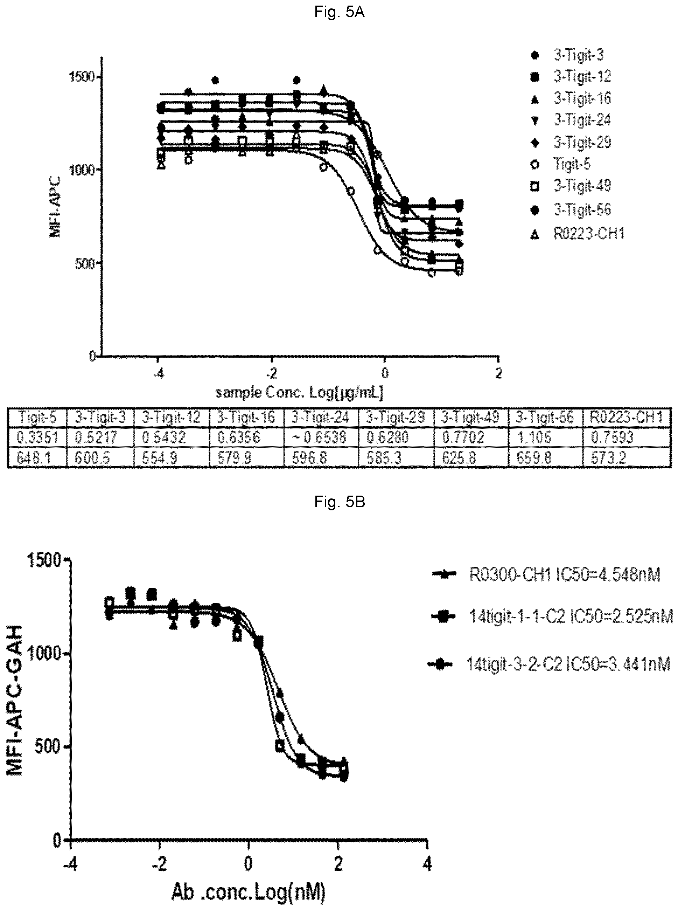

By means of result analysis, from results of Fig. 5A and Fig. 5B, it could be seen that, Tigit-5, 3Tigit-16, 3Tigit-24, 3Tigit-29, 14Tigit-1-1 and 14Tigit-3-2 can well block the binding of the tumor cells and the TIGIT, and the blocking effect is better than that of the control antibodies.

6. Detection the mouse monoclonal antibody blocking the binding of CHO-hCD112 and hTIGIT-hFc:

In order to verify that the anti-human TIGIT antibody blocks the binding of CHO-hCD112 and hTIGIT-hFc, the following experiments were specifically performed. The CHO-engineered cell CHO-hCD112 cells recombinantly expressing full-length human CD112 were counted; centrifugated at 300 g for 5 min; resuspended with an FCM buffer; and cell density was adjusted to 4E+06 cell/mL for later use. Samples to be detected (including a positive control antibody and a mouse monoclonal antibody) were diluted with the FCM buffer to 20μg/mL, and then the FCM buffer was used to perform 3x serial dilutions of 12 concentration points; an antigen protein hTIGIT-hFc was diluted with the FCM buffer to 120μg/mL; and an isotype control antibody was diluted with the FCM buffer to 20μg/mL. According to an experimental design, in each well, 50μL of the antigen protein and 50μL of the cell (the volume of each cell added into a 96-well V-shaped plate being 2E+05 cell/well) were respectively added in the 96-well V-shaped plate; and incubation was performed in dark on ice for 30 min. Centrifugation (300g/5min) was performed; supernatant was discarded; 200μL of the FCM buffer was added for washing once; centrifugation (300g/5min) was performed; and the supernatant was discarded. The antibody was added to the 96-well V-shaped plate (in 100μL/well); and incubation was performed in dark on ice for 30 min. Centrifugation (300g/5min) was performed; supernatant was discarded; 200μL of the FCM buffer was added for washing once; centrifugation (300g/5min) was performed; and the supernatant was discarded. Secondary antibodies (APC anti-human IgG Fc, 1:500 dilution) were added to the 96-well V-shaped plate (in 100μL/well); and incubation was performed in dark on ice for 30 min. Centrifugation (300g/5min) was performed; supernatant was discarded; 200μL of the FCM buffer was added for washing once; centrifugation (300g/5min) was performed; and the supernatant was discarded. 100μL of the 1×PBS was added for resuspending, and on-machine detection was performed.

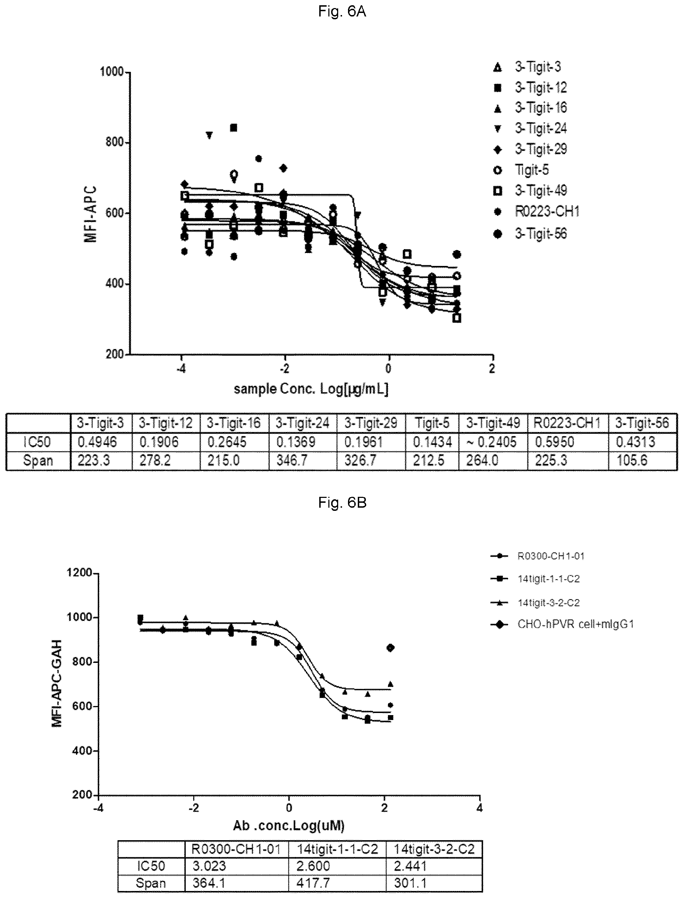

By means of result analysis, from results of Fig. 6A and Fig. 6B, it could be seen that, Tigit-5, 3Tigit-16, 3Tigit-24, 3Tigit-29, 14Tigit-1-1 and 14Tigit-3-2 can well block the binding of CHO-CD112 and the TIGIT, and the blocking effect is better than that of the control antibodies.

To sum up, the mouse monoclonal antibody obtained by means of screening may relatively better bind CHO-hTIGIT and PBMC in healthy people, and can relatively better block the binding of a TIGIT receptor on cells such as CHO-hTIGIT, CHO-hPVR, CHO-hCD112 and US-OS and a ligand PVR (the CHO-hPVR was to block the binding of the PVR ligand and the receptor TIGIT on cells).

**Embodiment 3 Species Cross-Binding Ability of Anti-human TIGIT antibody**

Detection of the mouse monoclonal antibody binding CHO-cynoTIGIT:

In order to verify that the anti-human TIGIT antibody binds to a monkey TIGIT, the following experiments were specifically performed. The CHO-engineered cell CHO-cynoTIGIT cells recombinantly expressing full-length human cynoTIGIT were counted; centrifugated at 300 g for 5 min; resuspended with an FCM buffer; and cell density was adjusted to 4E+06 cell/mL for later use. Samples to be detected (including a positive control antibody and a mouse monoclonal antibody) were diluted with the FCM buffer to 20μg/mL, and then the FCM buffer was used to perform 3x serial dilutions of 12 concentration points; and an isotype control antibody was diluted with the FCM buffer to 20μg/mL. According to an experimental design, in each well, 50μL of the antibody and 50μL of the cell (the volume of each cell added into a 96-well V-shaped plate being 2E+05 cell/well) were respectively added in the 96-well V-shaped plate; and incubation was performed in dark on ice for 30 min. Centrifugation (300g/5min) was performed; supernatant was discarded; 200μL of the FCM buffer was added for washing once; centrifugation (300g/5min) was performed; and the supernatant was discarded. Secondary antibodies (PE Goat anti-mouse IgG, 1:500 dilution) were added to the 96-well V-shaped plate (in 100μL/well); and incubation was performed in dark on ice for 30 min. Centrifugation (300g/5min) was performed; supernatant was discarded; 200μL of the FCM buffer was added for washing once; centrifugation (300g/5min) was performed; and the supernatant was discarded. 100μL of the 1×PBS was added for resuspending, and on-machine detection was performed.

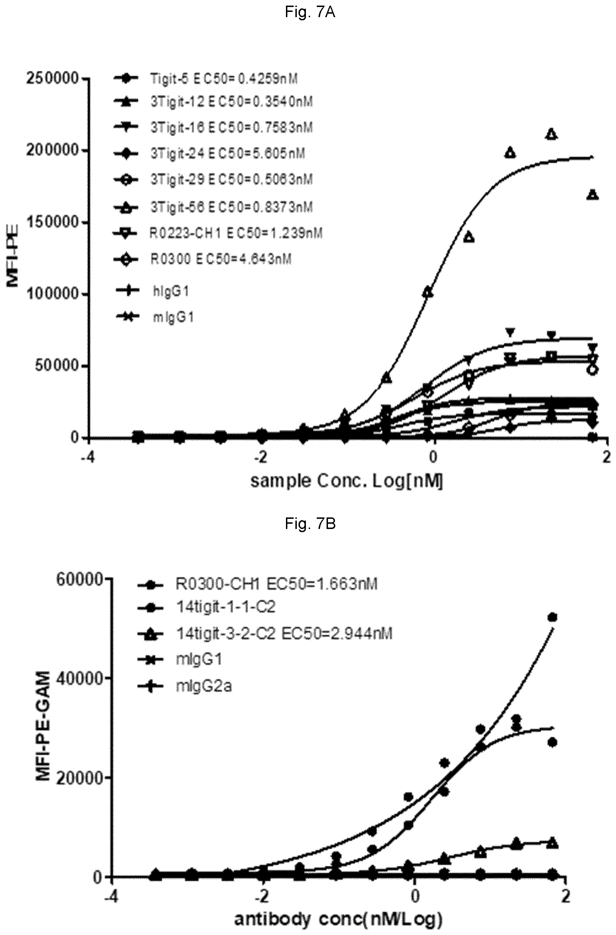

By means of result analysis, from results of Fig. 7A and Fig. 7B, it could be seen that, Tigit-5, 3Tigit-16, 3Tigit-29 and 14Tigit-1-1 can well bind to cynoTIGIT, and the binding effect is better than that of the control antibodies.

**Embodiment 4 Analysis of Binding Kinetics KD of Anti-human Tigit Antibody**

Binding kinetic constant analysis was performed on an anti-hTigit antibody and hTigit with an MD ForteBIO QKe platform. An experimental method included: a human Tigit extracellular domain recombinant protein hTigit-his (self-produced) carrying a his tag was diluted with an equilibration buffer (1×PBS+0.02% Tween 20), until a final concentration is 3μg/ml; the anti-hTigit antibody was diluted with the equilibration buffer (1×PBS+0.02%Tween 20) in 2x multiple proportions, an initial concentration being 10μg/ml, there being 7 concentrations in total; after an anti-Penta-HIS biosensor (ForteBIO, Cat.18-5122) was fully hydrated, first the hTigit-his recombinant protein was solidified for 120 seconds, equilibration was performed for 90 seconds, and then bound to the anti-hTigit antibody, binding time being 300 seconds, and dissociation time being 600 seconds; the whole reaction was performed at 25°C and 1000rpm; and finally, curve fitting was performed with Octet analysis software, and a binding kinetic constant KD of the anti-hTigit antibody was obtained.

As shown in Table 1, the binding kinetic constants KD of the anti-human TIGIT antibody and the human Tigit recombinant protein were all at a pM level.

Table 1

| Antibody name | KD, $\times$10E-10(M) |
| --- | --- |
| R0300-CH1 | 0.631 |
| Tigit-5 | 0.605 |
| 3Tigit-12 | 1.19 |
| 3Tigit-16 | 2.02 |
| 3Tigit-24 | 1.93 |
| 3Tigit-29 | 1.55 |
| 14Tigit-1-1 | 1.72 |
| 14Tigit-3-2 | 0.627 |

**Embodiment 5 Experiment on Function of Anti-TIGIT Antibody to Activate NK Cells**

In vitro functional screening was performed on the foregoing antibody with a 96-well real-time unlabeled cell analyzer. Most of cell detection methods used a traditional endpoint method, which only gave a final result and often needed to label and damage cells. These methods were unable to obtain real states of the cells during growing, and also unable to dynamically monitor and analyze the growth process of the cells. The real-time unlabeled cell analyzer uses a non-invasive method to record real-time growth states of the cells. Such imaging method was called "Live Content Imaging", which expanded the approach of recording and understanding cell growth, cell behaviors and cell morphology during experiments.

In this embodiment, the ability of the mouse monoclonal antibody to activate NK cells was detected with Agilent Biological RTCA SP; and the ability of the mouse monoclonal antibody to activate the NK cells was achieved by blocking the binding of the TIGIT on NK cell surfaces and PVR on target cell surfaces. A specific experimental process included the following. 1640 culture medium of 50μL/well was first added in a 96-well plate where an experiment was to be done, so as to perform an instrument automatic calibration stage; and subcultured U2OS cells (naturally and highly expressing the PVR) were digested with a TE buffer, and cell suspension obtained by means of digestion was resuspended with the 1640 culture medium and then counted:

$$U2OS\text{-}P4T2\text{-}1640\ 7.31{\times}10^5 cell/mL\ 99.14\%\ 16.07\mu m$$

The cell suspension was adjusted to $2{\times}10^5$cell/mL with the 1640 culture medium according to required cell numbers; then the cells were added in an experimental well plate in the volume of 50μL/well; standing was performed for 15 min in a biosafety cabinet, then the well plate covered with the cells was placed in an instrument, and culture was performed overnight. After 20h, after the U2OS was tightly attached to the bottom of the cell plate, antibodies and NK92-hTIGIT cells (recombinant expression of hTIGIT) started to be prepared. The subcultured NK92-TIGIT cells were blown into single cell suspensions, resuspended with the 1640 culture medium, and then counted:

$$NK92\text{-}TIGIT\text{-}1640\ 1.3{\times}10^5 cell/mL\ 45.98\%\ 12.32\mu m$$

The cell suspension was taken, and the number of the NK92-TIGIT cells was adjusted to $1{\times}10^5$cell/mL; and at the same time, the mouse monoclonal antibody to be detected was diluted to a required concentration, then the antibodies and the NK92-TIGIT cells were respectively added into the corresponding well plate in the volume of 50μL/well

according to the experimental design, and the blank group was supplemented with corresponding volume of 1640 culture medium.. A cell culture plate was placed in the biosafety cabinet to perform standing for 30 min, and at the same time, instrument parameters were adjusted for normalization processing; and the culture plate was continued to be placed in a detection clamping groove, and culture was continued in an incubator, and recording was performed.

After a parameter cell index in the instrument reached to a bottom value, the experiment was stopped, data was analyzed, and the killing rate of the cells was calculated according to the following formula:

$$\text{Cytotoxicity} = (\text{index E+T} - \text{index Exp})/\text{index E+T} \times 100\%$$

Plotting was performed with time as an X axis and killing efficiency as a Y axis.

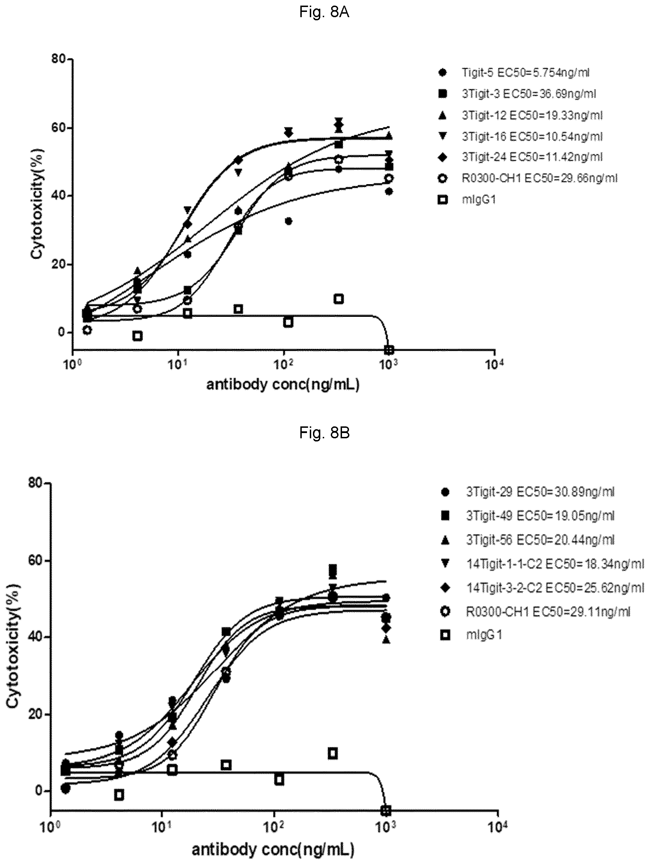

By means of result analysis, from results of Fig. 8A and Fig. 8B, it could be seen that, the mouse monoclonal antibody had good ability of activating the killing ability of NK92-hTIGIT; and the activation effect of Tigit-5, 3Tigit-12, 3Tigit-16 and 3Tigit-24 on the killing function of NK92-hTIGIT was obviously better than that of a control sample.

**Embodiment 6 Experiment on Activation of Killing Function of NK Cells by Anti-TIGIT Antibody**

A double fluorescence method was used to further detect the activation of the killing function of NK cells by an anti-TIGIT antibody:

Target cell planking

TE digestion cultured U2OS cells were counted, and a sufficient number of cells for the experiment was taken. The cells were well mixed with 10mL of PBS, centrifugation was performed at 400g/5min, and then supernatant was discarded. The cells were resuspended with 2 mL, 2μL of CFSE was added, and the cells were dyed for 15min at 37°C. The cells were resuspended by replenishing 8mL of the PBS. Centrifugation was performed on the cells at 400g/5min, and then the cells were resuspended again with 10mL of the PBS. Centrifugation was performed at 400g/5min, the supernatant was discarded, and the cells were resuspended with an appropriate 10%FBS 1640 culture medium. The cells were counted again. The number of the cells was adjusted to $2 \times 10^5$/mL with the culture medium, and the cells with the volume of 100μL were added in the 96-well plate (there were 60 wells in the middle, and edges were sealed with 100μL of the PBS). The cells were continued to be cultured for 20h.

Effector cells and antibodies were added.

After 20h, after the U2OS was tightly attached to the bottom of the cell plate, the antibodies and NK92-TIGIT cells started to be prepared. The antibodies to be detected and isotype hlgG1 were diluted to four times of the desired concentration. The subcultured NK92-TIGIT cells were blown into single cell suspensions, resuspended with the 1640 culture medium, and then counted. The cell suspension was taken, and the number of the NK92-TIGIT cells was adjusted to $4 \times 10^5$cell/mL. The antibodies and the NK92-TIGIT cells were respectively added into the corresponding well plate in the volume of 50μL/well according to the experimental design, and the blank group was supplemented with corresponding volume of 1640 culture medium.. Culture was continued in the incubator.

Flow detection

The 96-well plate was taken after 16h, cell supernatant was transferred to another 96-well plate, taking care to correspond one to the other; and 35μL of the PBS was added to wash the bottom of the plate, and cleaning fluid was transferred into the corresponding 96-well plate. 35μL of 0.25%Trypsin-EDTA was added in each well of the 96-well plate, and placed in a 37°C incubator for about 5 min. The liquid in the previous 96-well plate was transferred into the current digested 96-well plate in a one-to-one correspondence, digestion was terminated, and the cells were gently blown over. 10μL of a PI solution was added in each well, so as to cause the final concentration to be 1μg/mL, and well mixing was gently performed (remember to immediately perform on-machine detection, the time should not exceed 45 min). A flow cytometer was used to analyze the cells in each well.

Data analysis

CFSE and PI double-positive cells were killed cells, and CFSE single-positive cells were considered as the sum of all target cells. Therefore, the absolute killing efficiency of antibodies was Cytotoxicity=(the number of PI and CFSE double-positive cells)/(the number of CFSE single-positive cells)×100%; test cells Cytotoxicity(%) with different concentrations added to an antibody group were first calculated, and T+E wells were used as negative control wells; and then relative cell killing efficiency=(experiment group-control wells)/control wells×100% was calculated. GraphPad Prism 5 software used an antibody concentration as a horizontal ordinate and the value of the calculated relative killing efficiency Cytotoxicity(%) as a longitudinal coordinate, to perform nonlinear fitting, and an $EC_{50}$ value was calculated.

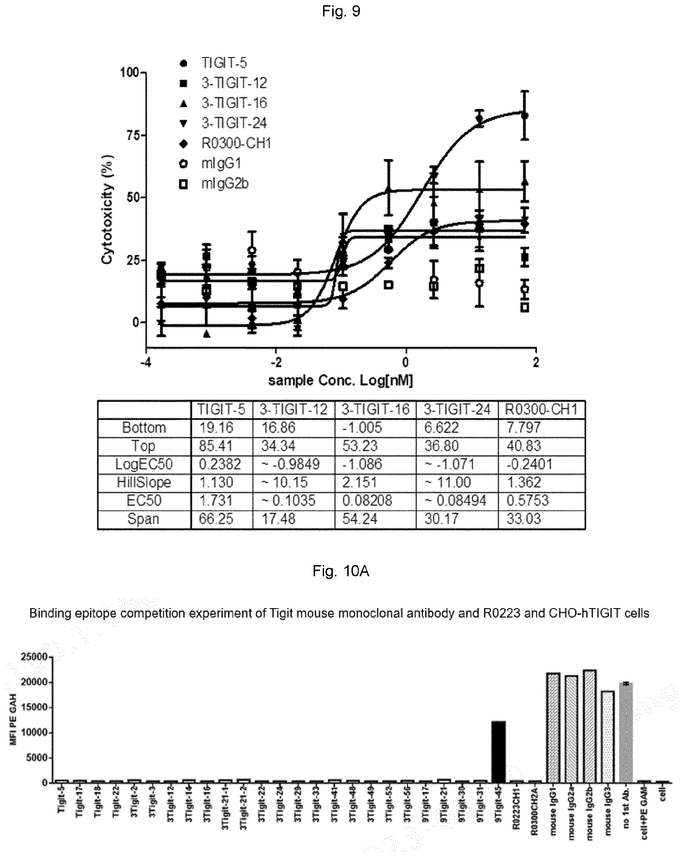

By means of result analysis, from results of Fig. 9, it could be seen that, Tigit-5 and 3Tigit-16 can activate the ability of NK92-hTIGIT to kill U2-OS, and the activation effect is obviously better than that of the control sample.

**Embodiment 7 Analysis of Competitive Epitope of Anti-human Tigit Antibody**

1. epitope binning

Epitope competition analysis was performed on an anti-human TIGIT antibody with an MD ForteBIO QKe platform. An experimental method included: a human Tigit extracellular domain recombinant protein hTigit-his (Acrobiosystem, Cat.TIT-H2H3, lot-1336-76GF1-F9) carrying a his tag was diluted with an equilibration buffer (1×PBS+0.02%Tween 20), until the final concentration was 1.5μg/ml. The anti-human Tigit antibody was diluted with the equilibration buffer (1×PBS+0.02%Tween 20), the final concentration being 5 μg/ml. After an anti-Penta-HIS biosensor (ForteBIO, Cat. 18-5122) was fully hydrated, first the hTigit-his recombinant protein was solidified for 180 seconds, then equilibrated for 30 seconds, and bound to the first anti-human Tigit antibody for 180 seconds, then equilibrated for 30 seconds, and then bound to the second anti-human Tigit antibody, binding time being 300 seconds. The whole reaction was performed at 25°C and 1000rpm; and finally, Octet analysis software was used to perform epitope binning analysis.

As shown in Table 2 and Table 3, the epitope of one anti-human Tigit antibody (9Tigit-45) was different from that of R0223. The epitope of 15 anti-human Tigit antibodies (Tigit-5, Tigit-17, Tigit-18, Tigit-22, 3Tigit-2, 3Tigit-14, 3Tigit-21-1, 3Tigit-21-2, 3Tigit-22, 3Tigit-33, 3Tigit-41, 3Tigit-56, 9Tigit-17, 9Tigit-21, and 9Tigit-45) were different from that of R0300.

Table 2

| Tigit-5 | Tigit-17 | Tigit-18 | Tigit-22 | 3Tigit-2 | 3Tigit-3 | 3Tigit-9 |
|---|---|---|---|---|---|---|
| 0.0443 | 0.047 | 0.0383 | 0.0353 | 0.0118 | 0.0105 | 0.0165 |
| 3Tigit-12 | 3Tigit-14 | 3Tigit-16 | 3Tigit-21-1 | 3Tigit-21-2 | 3Tigit-22 | 3Tigit-24 |
| 0.01 | 0.0126 | 0.008 | 0.0133 | 0.0206 | 0.0583 | 0.0379 |
| 3Tigit-29 | 3Tigit-33 | 3Tigit-41 | 3Tigit-48 | 3Tigit-49 | 3Tigit-51 | 3Tigit-52 |
| -0.0018 | 0.0225/0.059 | 0.0108 | 0.0153 | 0.0413 | 0.0613 | 0.0307 |
| 3Tigit-56 | 6Tigit-10 | 8Tigit-4 | 8Tigit-7 | 9Tigit-17 | 9Tigit-21 | 9Tigit-30 |
| 0.0323 | 0.0341 | 0.026 | 0.0148 | 0.0395 | 0.0504 | 0.0537 |
| 9Tigit-31 | 9Tigit-45 | R0223 | | | | |
| 0.0484 | 0.3347 | 0.0566 | | | | |

Table 3

| Tigit-5 | Tigit-17 | Tigit-18 | Tigit-22 | 3Tigit-2 | 3Tigit-3 | 3Tigit-9 |
|---|---|---|---|---|---|---|
| 0.6679 | 0.6297 | 0.5439 | 0.6136 | 0.2683 | 0.0198 | 0.0109 |
| 3Tigit-12 | 3Tigit-14 | 3Tigit-16 | 3Tigit-21-1 | 3Tigit-21-2 | 3Tigit-22 | 3Tigit-24 |
| 0.0196 | 0.3038 | 0.0157 | 0.3001 | 0.3142 | 0.8526 | 0.0132 |
| 3Tigit-29 | 3Tigit-33 | 3Tigit-41 | 3Tigit-48 | 3Tigit-49 | 3Tigit-51 | 3Tigit-52 |
| 0.0254 | 0.9022 | 0.295 | 0.0035 | 0.032 | 0.0165 | 0.0166 |
| 3Tigit-56 | 6Tigit-10 | 8Tigit-4 | 8Tigit-7 | 9Tigit-17 | 9Tigit-21 | 9Tigit-30 |
| 0.4351 | 0.0124 | 0.015 | 0.0161 | 0.5831 | 0.6536 | 0.0238 |
| 9Tigit-31 | 9Tigit-45 | R0223 | R0300 | | | |
| 0.0289 | 0.8743 | 0.0687 | 0.0316 | | | |

2. Flow analysis of antibody epitope competition at cellular level

The anti-Tigit antibody was diluted with 1×FCM buffer (1×FBS+3%BSA), the first antibody included benchmarking antibodies R0223 and R0300 (hIgG1 subtype), and a hIgG1 isotype control antibody, with a concentration being 10μg/ml, and the second antibody included the anti-human Tigit antibody, benchmarking antibodies R0223-CH1 and R0300-CH1(m!gG1 subtype), and a mIgG1 isotype control antibody, with a concentration being 1μg/ml. The CHO stable cell strains recombinantly expressing human Tigit were resuspended with 1×FCM buffer, cell density was adjusted to be 2×10E6/ml; and the cells were spread in the 96-well plate in 100μl/well. Centrifugated for 5 min, then the supernatant was discarded in a sucking manner, 100μl of the first antibody was added, incubated on ice for 30 min, and then 100μl of the second antibody was added, and incubated on ice for 30 min. After centrifugated at 250×g for 5 min, the supernatant was discarded in a sucking manner. After washed for 2 times with the 1×FCM, PE-labeled goat anti-mouse Fc fluorescent secondary antibodies diluted with the 1×FCM buffer were added in 100μl/well (dilution ratio being 1:500) (Biolegend, Cat.405307); and incubated on ice for 30 min. After centrifugated at 250×g for 5 min, the supernatant was discarded in a sucking manner. After washed for 2 times with the 1×FCM, the cells were resuspended by adding the 1×PBS in 100μl/well; and the flow cytometer (Beckman, cytoFLEX) was used for detection. On the contrary, the first antibody included the anti-human Tigit antibody, the benchmarking antibodies R0223-CH1 and R0300-CH1(m!gG1 subtype), and the mIgG1 isotype control antibody, with the concentration being 10μg/ml; and the second antibody included the benchmarking antibodies R0223 and R0300 (hIgG1 subtype), and the hIgG1 isotype control antibody, with the concentration being 1μg/ml. The fluorescent secondary antibodies were the PE-labeled goat anti-mouse Fc fluorescent secondary antibodies (dilution ratio being 1:500) (Biolegend, Cat.409304).

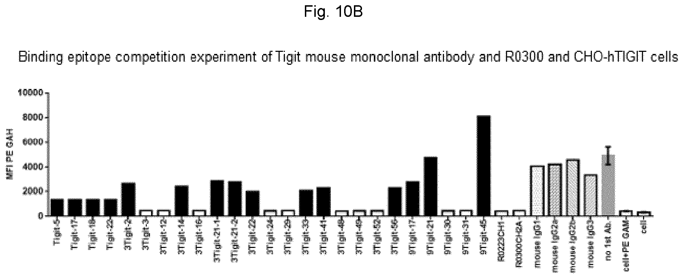

As shown in Fig. 10A and Fig. 10B, results were the same as step 1 in Embodiment 7, and the epitope of one anti-human Tigit antibody was different from that of R0223. The epitope of 15 anti-human Tigit antibodies were different from that of R0300, and the R0223 epitope overlaps with the R0300 epitope.

Antibody sequences involved in the present disclosure are shown as follows.

Sequences with underlines are CDR sequences, sequences without underlines were FR sequences, and numbers in parentheses represent sequence numbers.

VH sequence of a Tigit-5 mouse monoclonal antibody:

QVQLQQPGAELVRPGASVKLSCKASGYSFT(43)<u>SYWMN(1)</u>WVKQRPGQGLEWIG(44)<u>MIRP SDSETRLNQMFKD(2)</u>KATLTVDKSSSTAYMQLSSPTSDDSAVYYCAG(45)<u>IHDYGHGAY(3)</u>WGQG

TLVTVSA(46).

VL sequence of a Tigit-5 mouse monoclonal antibody:

DIQMIQSPASLSVSVGETVTITC(71)<u>RASENIYSNLA(22)</u>WYQQKQGKSPQLLVY(72)<u>AASHLP D(23)</u>GVPSRFSGSGSGTQYSLKINSLQSEDFGSYYC(73)<u>QHFWGTPRT(24)</u>FGGGTKLEIKR(74).

VH sequence of a 3Tigit-16 mouse monoclonal antibody:

EVQLQQSGPELVKPGASVKMSCKASGFTFT(47)<u>DYFMD(4)</u>WVKQSRGASFEWIG(48)<u>RLNP NNGRTSYNQKFKG(5)</u>KATLTVDKSSSTAYMELNSLTSEDSAVYYCAR(49)<u>GDLGRWYFDV(6)</u>WGA GTTVTVSS(50).

VL sequence of a 3Tigit-16 mouse monoclonal antibody:

QIVLTQSPAIMSASPGEKVTMTC(75)<u>SASSSVSYMH(25)</u>WYQQKSGTSPKRWIY(76)<u>DTSKLA S(26)</u>GVPTRFSGSGSGTSYSLTISSMEAEDAASYFC(77)<u>QQWSSNSLT(27)</u>FGAGTKLELKR(78).

VH sequence of a 3Tigit-24 mouse monoclonal antibody:

QVQLQQSGAELMKPGASVKISCKATGYTFS(51)<u>SYWIE(7)</u>WVKQRPGHGLEWIG(52)<u>EILSGS GRTYFNEKFKG(8)</u>KATFTADTSSNTSYMQLSSLTSEDSAVYFCAR(53)<u>RGLRGPYYFDY(9)</u>WGQG TTLTVSS(54).

VL sequence of a 3Tigit-24 mouse monoclonal antibody:

DIVLTQSPASLAVSLGQRATISY(79)<u>RASKSVSTSGYSYMH(28)</u>WNQQKPGQPPRLLIY(80)<u>LV SNLES(29)</u>GVPARFSGSGSGTDFTLNIHPVEEEDAATYYC(81)<u>QHIRELTR(30)</u>SGGGTKLEIKR(82) .

VH sequence of a 3Tigit-29 mouse monoclonal antibody:

EVQLQQSGPELVKPGASVKISCKASGYTFT(55)<u>DYNMH(10)</u>WVKQSLGKSLEWIG(56)<u>YVNSF NGNTGYSQKFKS(11)</u>KVTLTVDRSSRTAYMDLRSLTSEDSAVFYCAR(57)<u>SSRGGFAY(12)</u>WGQG TLVTVSA(58).

VL sequence of a 3Tigit-29 mouse monoclonal antibody:

QIVLTQSPAIMSASPGEKVTMTC(83)<u>SASSSVSYMH(31)</u>WYQQKSGTSPKRWIY(84)<u>DTSKLA S(32)</u>GVPARFSGSVSGTSYSLTISSMEAEDAATYYC(85)<u>QQWSSNSLT(33)</u>FGAGTKLELKR(86).

VH sequence of a 14Tigit-1-1 mouse monoclonal antibody:

DVQLQESGPGLVKPSQSLSLTCTVTGYSIT(59)<u>SDYAWN(13)</u>WIRQFPGNKLEWMG(60)<u>YISY SGSTRSNPSLKS(14)</u>RISITRDTSKNQFFLQLNSMTAEDTATYYCGG(61)<u>WEVRNYYAMDY(15)</u>WG QGTSVTVSS(62).

VL sequence of a 14Tigit-1-1 mouse monoclonal antibody:

DIVMTQSHKFMSTSVGDRVSITC(87)<u>KASQHVSTAVA(34)</u>WYQQKPGQSPKLLIY(88)<u>SASYRY T(35)</u>GVPDRFTGSGSGTDFTFTISSVQAEDLAVYYC(89)<u>QQHYNTPWT(36)</u>FGGGTKLEIKR(90).

VH sequence of a 14Tigit-3-2 mouse monoclonal antibody:

EVQLQQSGAELVKPGASVKLSCTASGFNIK(63)<u>DTYIH(16)</u>WVKQRPDQGLEWIG(64)<u>GIGPA NGNTKFDPKFQG(17)</u>KATITADTSSNTAYLQLSGLTSEDTAVYYCAK(65)<u>LLLRFYVLAY(18)</u>WGQG TSVTVSS(66).

VL sequence of a 14Tigit-3-2 mouse monoclonal antibody:

DIVLTQSPASLAVSLGQRATISY(91)<u>RASKSVSTSGYSYMH(37)</u>WNQQKPGQPPRLLIY(92)<u>LV SNLES(38)</u>GVPARFSGSGSGTDFTLNIHPVEEEDAATYYC(93)<u>QHIRELTR(39)</u>SGGGTKLEIKR(94) .

VH sequence of a 3Tigit-12 mouse monoclonal antibody:

QVQLQQSGAELVRPGTSVKMSCKAAGYTFT(67)<u>NYWIG(19)</u>WVKQRPGHGLEWIG(68)<u>DIYP GGGYTNYDEKFKG(20)</u>KATLTADTSSRTAYMQLSSLTSEDSAIYHCVN(69)<u>GGYGSTYGYFDV(21)</u> WGAGTTVTVSS(70).

VL sequence of a 3Tigit-12 mouse monoclonal antibody:

DIVMSQSPSSLPVSVGEKVTMSC(95)<u>KSSQSLLFSSDQKNYLA(40)</u>WYQQKPGQSPKLLIY(96)<u>WASTRES(41)</u>GVPDRLTGSGSGTDFTLTISSVKAEDLAVYYC(97)<u>QQYHKYPFT(42)</u>FGGGTKLEIKR(98).

Various technical features of the above embodiments may be combined arbitrarily. For brevity of description, description is not made to all possible combinations of the various technical features of the above embodiments are described. However, all the combinations of these technical features should be considered to fall within the scope of disclosure contained in the specification as long as there is no contradiction between the combinations of those technical features.

The above embodiments merely illustrate several implementations of the present disclosure, which are specifically described in detail, but are not to be construed as limiting the scope of the present patent for the present disclosure. It should be pointed out that those of ordinary skill in the art can also make some modifications and improvements without departing from the concept of the present disclosure, and these modifications and improvements all fall within the scope of protection of the present disclosure. Accordingly, the scope of the patent of the present disclosure should be subject to the appended claims.

**Claims**

1.  An antibody specifically recognizing a T cell Ig and ITIM domain (TIGIT) or an antigen-binding fragment thereof, wherein heavy chain CDR1, CDR2 and CDR3 are selected from at least one of combinations of SEQ ID NOs:1-3, SEQ ID NOs:4-6, SEQ ID NOs:7-9, SEQ ID NOs:10-12, SEQ ID NOs:13-15, SEQ ID NOs:16-18, or SEQ ID NOs:19-21;

    light chain CDR1, CDR2 and CDR3 are selected from at least one of SEQ ID NOs:22-24, SEQ ID NOs:25-27, SEQ ID NOs:28-30, SEQ ID NOs:31-33, SEQ ID NOs:34-36, SEQ ID NOs:37-39, or SEQ ID NOs:40-42;

    optionally, the antibody or the antigen-binding fragment thereof has at least one of the following CDR sequence combinations:

|  | H-CDR1 | H-CDR2 | H-CDR3 | L-CDR1 | L-CDR2 | L-CDR3 |
|---|---|---|---|---|---|---|
| Tigit-5 | SEQ ID NO:1 | SEQ ID NO:2 | SEQ ID NO:3 | SEQ ID NO: 22 | SEQ ID NO: 23 | SEQ ID NO: 24 |
| 3Tigit-16 | SEQ ID NO:4 | SEQ ID NO:5 | SEQ ID NO:6 | SEQ ID NO: 25 | SEQ ID NO: 26 | SEQ ID NO: 27 |
| 3Tigit-24 | SEQ ID NO:7 | SEQ ID NO:8 | SEQ ID NO:9 | SEQ ID NO: 28 | SEQ ID NO: 29 | SEQ ID NO: 30 |
| 3Tigit-29 | SEQ ID NO: 10 | SEQ ID NO: 11 | SEQ ID NO: 12 | SEQ ID NO: 31 | SEQ ID NO: 32 | SEQ ID NO: 33 |
| 14Tigit-1-1 | SEQ ID NO: 13 | SEQ ID NO: 14 | SEQ ID NO: 15 | SEQ ID NO: 34 | SEQ ID NO: 35 | SEQ ID NO: 36 |
| 14Tigit-3-2 | SEQ ID NO: 16 | SEQ ID NO: 17 | SEQ ID NO: 18 | SEQ ID NO: 37 | SEQ ID NO: 38 | SEQ ID NO: 39 |
| 3Tigit-12 | SEQ ID NO: 19 | SEQ ID NO: 20 | SEQ ID NO: 21 | SEQ ID NO: 40 | SEQ ID NO: 41 | SEQ ID NO: 42 |

2.  The antibody or the antigen-binding fragment thereof according to claim 1, wherein heavy chain FR1, FR2, FR3 and FR4 are selected from at least one of combinations of SEQ ID NOs:43-46, SEQ ID NOs:47-50, SEQ ID NOs:51-54, SEQ ID NOs:55-58, SEQ ID NOs:59-62, SEQ ID NOs:63-66, or SEQ ID NOs:67-70;

    light chain FR1, FR2, FR3 and FR4 are selected from at least one of SEQ ID NOs:71-74, SEQ ID NOs:75-78, SEQ ID NOs:79-82, SEQ ID NOs:83-86, SEQ ID NOs:87-90, SEQ ID NOs:91-94, or SEQ ID NOs:95-98; optionally, the antibody or the antigen-binding fragment thereof further comprises at least one of the following framework region sequence combinations:

|  | H-FR1 | H-FR2 | H-FR3 | H-FR4 | L-FR1 | L-FR2 | L-FR3 | L-FR4 |
|---|---|---|---|---|---|---|---|---|
| Tigit-5 | SEQ ID NO:43 | SEQ ID NO:44 | SEQ ID NO:45 | SEQ ID NO:46 | SEQ ID NO:71 | SEQ ID NO:72 | SEQ ID NO: 73 | SEQ ID NO:74 |
| 3Tigit-16 | SEQ ID NO:47 | SEQ ID NO:48 | SEQ ID NO:49 | SEQ ID NO:50 | SEQ ID NO:75 | SEQ ID NO:76 | SEQ ID NO:77 | SEQ ID NO:78 |
| 3Tigit-24 | SEQ ID NO:51 | SEQ ID NO:52 | SEQ ID NO:53 | SEQ ID NO:54 | SEQ ID NO:79 | SEQ ID NO:80 | SEQ ID NO:81 | SEQ ID NO:82 |
| 3Tigit-29 | SEQ ID NO:55 | SEQ ID NO:56 | SEQ ID NO:57 | SEQ ID NO:58 | SEQ ID NO:83 | SEQ ID NO:84 | SEQ ID NO:85 | SEQ ID NO:86 |
| 14Tigit-1-1 | SEQ ID NO:59 | SEQ ID NO:60 | SEQ ID NO:61 | SEQ ID NO:62 | SEQ ID NO:87 | SEQ ID NO:88 | SEQ ID NO:89 | SEQ ID NO:90 |
| 14Tigit-3-2 | SEQ ID NO:63 | SEQ ID NO:64 | SEQ ID NO:65 | SEQ ID NO:66 | SEQ ID NO:91 | SEQ ID NO:92 | SEQ ID NO:93 | SEQ ID NO:94 |
| 3Tigit-12 | SEQ ID NO:67 | SEQ ID NO:68 | SEQ ID NO:69 | SEQ ID NO:70 | SEQ ID NO:95 | SEQ ID NO:96 | SEQ ID NO:97 | SEQ ID NO:98 |

3. The antibody or the antigen-binding fragment thereof according to claim 1 or 2, wherein the antigen-binding fragment is one of F(ab')2, Fab, scFv, or a bispecific antibody;

optionally, the antibody has a constant region, a heavy chain constant region sequence is selected from a constant region sequence of any one of IgG1, IgG2, IgG3, IgG4, IgA, IgM, IgE, or IgD, and a light chain constant region is a κ or a λ chain; and
optionally, the species source of the constant region is selected from cattle, horses, pigs, sheep, goats, rats, mice, dogs, cats, rabbits, camels, donkeys, deer, minks, chickens, ducks, geese, or humans.

4. A nucleic acid, encoding the antibody or the antigen-binding fragment thereof according to any of claims 1 to 3.

5. A vector comprising the nucleic acid according to claim 4.

6. A cell comprising the nucleic acid according to claim 4 or the vector according to claim 5.

7. A method for producing the antibody or the antigen-binding fragment thereof according to any of claims 1 to 3, comprising:

culturing the cell according to claim 6 in a culture medium; and
recovering an antibody or an antigen-binding fragment thereof produced from the culture medium or the cultured cell.

8. A pharmaceutical composition comprising the antibody or the antigen-binding fragment thereof according to any of claims 1 to 3, and one or more of pharmacologically acceptable excipients, diluents or carriers.

9. Use of the antibody or the antigen-binding fragment thereof according to any of claims 1 to 3 in preparation of a medicine for treating or preventing infectious diseases, immune diseases or tumors.

10. A kit comprising at least one of the following components:

i) the antibody or the antigen-binding fragment thereof according to any of claims 1 to 3, and an optional container used for holding the antibody or the antigen-binding fragment thereof; or
ii) the pharmaceutical composition according to claim 8, and an optional container used for holding the phar-

maceutical composition.

Fig. 1A

Fig. 1B

|  | R0223-CH1 | R0223 | R0300 | Tigit-5 | 3-Tigit-16 | 3-Tigit-24 | 3-Tigit-29 |
|---|---|---|---|---|---|---|---|
| EC50 | 0.6219 | 0.9754 | 1.187 | 0.2557 | 0.2943 | 0.3370 | 0.3096 |
| Span | 31525 | 12295 | 9409 | 21023 | 34625 | 112661 | 30638 |

Fig. 1C

Fig. 2A

| | R0223-CH1 | 3-Tigit-16 | 3-Tigit-24 | 3-Tigit-29 |
|---|---|---|---|---|
| EC50 | 0.1263 | 0.02474 | 0.03340 | 0.02173 |
| Span | 1191 | 921.5 | 2207 | 687.2 |

Fig. 2B

| | R0223-CH1 | Tigit-5 |
|---|---|---|
| EC50 | 0.1263 | ~ 309.9 |
| Span | 1191 | ~ 107674 |

Fig. 2C

Fig. 3A

Fig. 3B

## CHO-hTIGIT blocking IC$_{50}$ by FACS

Fig. 4A

Fig. 4B

Fig. 5A

| Tigit-5 | 3-Tigit-3 | 3-Tigit-12 | 3-Tigit-16 | 3-Tigit-24 | 3-Tigit-29 | 3-Tigit-49 | 3-Tigit-56 | R0223-CH1 |
|---------|-----------|------------|------------|------------|------------|------------|------------|-----------|
| 0.3351 | 0.5217 | 0.5432 | 0.6356 | ~ 0.6538 | 0.6280 | 0.7702 | 1.105 | 0.7593 |
| 648.1 | 600.5 | 554.9 | 579.9 | 596.8 | 585.3 | 625.8 | 659.8 | 573.2 |

Fig. 5B

Fig. 6A

| | 3-Tigit-3 | 3-Tigit-12 | 3-Tigit-16 | 3-Tigit-24 | 3-Tigit-29 | Tigit-5 | 3-Tigit-49 | R0223-CH1 | 3-Tigit-56 |
|---|---|---|---|---|---|---|---|---|---|
| IC50 | 0.4946 | 0.1906 | 0.2645 | 0.1369 | 0.1961 | 0.1434 | ~ 0.2405 | 0.5950 | 0.4313 |
| Span | 223.3 | 278.2 | 215.0 | 346.7 | 326.7 | 212.5 | 264.0 | 225.3 | 105.6 |

Fig. 6B

| | R0300-CH1-01 | 14tigit-1-1-C2 | 14tigit-3-2-C2 |
|---|---|---|---|
| IC50 | 3.023 | 2.600 | 2.441 |
| Span | 364.1 | 417.7 | 301.1 |

Fig. 7A

Fig. 7B

Fig. 8A

- Tigit-5 EC50=5.754ng/ml
- 3Tigit-3 EC50=36.69ng/ml
- 3Tigit-12 EC50=19.33ng/ml
- 3Tigit-16 EC50=10.54ng/ml
- 3Tigit-24 EC50=11.42ng/ml
- R0300-CH1 EC50=29.66ng/ml
- mIgG1

Fig. 8B

- 3Tigit-29 EC50=30.89ng/ml
- 3Tigit-49 EC50=19.05ng/ml
- 3Tigit-56 EC50=20.44ng/ml
- 14Tigit-1-1-C2 EC50=18.34ng/ml
- 14Tigit-3-2-C2 EC50=25.62ng/ml
- R0300-CH1 EC50=29.11ng/ml
- mIgG1

Fig. 9

| | TIGIT-5 | 3-TIGIT-12 | 3-TIGIT-16 | 3-TIGIT-24 | R0300-CH1 |
|---|---|---|---|---|---|
| Bottom | 19.16 | 16.86 | -1.005 | 6.622 | 7.797 |
| Top | 85.41 | 34.34 | 53.23 | 36.80 | 40.83 |
| LogEC50 | 0.2382 | ~ -0.9849 | -1.086 | ~ -1.071 | -0.2401 |
| HillSlope | 1.130 | ~ 10.15 | 2.151 | ~ 11.00 | 1.362 |
| EC50 | 1.731 | ~ 0.1035 | 0.08208 | ~ 0.08494 | 0.5753 |
| Span | 66.25 | 17.48 | 54.24 | 30.17 | 33.03 |

Fig. 10A

Binding epitope competition experiment of Tigit mouse monoclonal antibody and R0223 and CHO-hTIGIT cells

Fig. 10B

Binding epitope competition experiment of Tigit mouse monoclonal antibody and R0300 and CHO-hTIGIT cells

## INTERNATIONAL SEARCH REPORT

| International application No. |
| --- |
| **PCT/CN2021/131743** |

### A.   CLASSIFICATION OF SUBJECT MATTER

C07K 16/28(2006.01)i;  C12N 15/13(2006.01)i;  A61K 39/395(2006.01)i;  A61P 35/00(2006.01)i

According to International Patent Classification (IPC) or to both national classification and IPC

### B.   FIELDS SEARCHED

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

WPABSC; CNTXT; CNABS; OETXT; WPABS; ENTXT; CJFD; DWPI; ENTXTC; VEN; GenBank; China Patents Biological Sequence Search System; PUBMED; WANFANG: TIGIT, VSIG9, WUCAM, VSTM3, 抗体, CD155, 肿瘤, NK细胞, antibody, tumor, NK cells, CDRs, SEQ ID NO: 1-42

### C.   DOCUMENTS CONSIDERED TO BE RELEVANT

| Category* | Citation of document, with indication, where appropriate, of the relevant passages | Relevant to claim No. |
| --- | --- | --- |
| A | CN 109384846 A (HEFEI RUIDA IMMUNE DRUGS RESEARCH INSTITUTE CO., LTD.) 26 February 2019 (2019-02-26) claims 1-15, description table 1, sequences | 1-10 |
| A | CN 110352200 A (I-MAB) 18 October 2019 (2019-10-18) claims 1-40, description embodiment 1 | 1-10 |
| A | CN 111748580 A (BIO-THERA SOLUTIONS, LTD.) 09 October 2020 (2020-10-09) entire document | 1-10 |
| A | WO 2019137548 A1 (NANJING LEGEND BIOTECH CO., LTD.) 18 July 2019 (2019-07-18) entire document | 1-10 |
| A | US 2020255516 A1 (JIANGSU HENGRUI MEDICINE CO., LTD. et al.) 13 August 2020 (2020-08-13) entire document | 1-10 |
| A | WO 2016106302 A1 (BRISTOL-MYERS SQUIBB COMPANY) 30 June 2016 (2016-06-30) entire document | 1-10 |
| A | US 2018371083 A1 (MERCK SHARP & DOHME CORP.) 27 December 2018 (2018-12-27) entire document | 1-10 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **18 January 2022** | **26 January 2022** |

| Name and mailing address of the ISA/CN | Authorized officer |
| --- | --- |
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

| INTERNATIONAL SEARCH REPORT | International application No. |
| --- | --- |
| | **PCT/CN2021/131743** |

**C.    DOCUMENTS CONSIDERED TO BE RELEVANT**

| Category* | Citation of document, with indication, where appropriate, of the relevant passages | Relevant to claim No. |
| --- | --- | --- |
| A | WO 2019129261 A1 (BEIGENE, LTD.) 04 July 2019 (2019-07-04) entire document | 1-10 |
| A | US 2018185480 A1 (YISSUM RESEARCH DEVELOPMENT COMPANY OF THE HEBREW UNIVERSITY OF JERUSALEM LTD. et al.) 05 July 2018 (2018-07-05) entire document | 1-10 |
| A | YANG, F. et al. "A Cross-Species Reactive TIGIT-Blocking Antibody Fc Dependently Confers Potent Antitumor Effects" *THE JOURNAL OF IMMUNOLOGY*, Vol. vol. 205, 04 September 2020 (2020-09-04), pp. 2156-2168 | 1-10 |

Form PCT/ISA/210 (second sheet) (January 2015)

**EP 4 238 991 A1**

| INTERNATIONAL SEARCH REPORT | International application No. |
|---|---|
| | **PCT/CN2021/131743** |

**Box No. I**      **Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

   a.  ☑ forming part of the international application as filed:

       ☑ in the form of an Annex C/ST.25 text file.

       ☐ on paper or in the form of an image file.

   b.  ☐ furnished together with the international application under PCT Rule 13*ter*.1(a) for the purposes of international search only in the form of an Annex C/ST.25 text file.

   c.  ☐ furnished subsequent to the international filing date for the purposes of international search only:

       ☐ in the form of an Annex C/ST.25 text file (Rule 13*ter*.1(a)).

       ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

| INTERNATIONAL SEARCH REPORT<br>Information on patent family members | | | | International application No.<br><br>**PCT/CN2021/131743** |

| Patent document<br>cited in search report | | | Publication date<br>(day/month/year) | Patent family member(s) | | | Publication date<br>(day/month/year) |
|---|---|---|---|---|---|---|---|
| CN | 109384846 | A | 26 February 2019 | None | | | |
| CN | 110352200 | A | 18 October 2019 | CA | 3086936 | A1 | 15 August 2019 |
| | | | | PH | 12020550650 | A1 | 19 April 2021 |
| | | | | JP | 2021510141 | A | 15 April 2021 |
| | | | | ZA | 202003892 | B | 27 October 2021 |
| | | | | SG | 11202004521 Q | A | 29 June 2020 |
| | | | | IL | 276257 | D0 | 30 September 2020 |
| | | | | EP | 3568415 | A1 | 20 November 2019 |
| | | | | EP | 3568415 | A4 | 06 January 2021 |
| | | | | WO | 2019154415 | A1 | 15 August 2019 |
| | | | | KR | 20200054133 | A | 19 May 2020 |
| | | | | KR | 102224556 | B1 | 09 March 2021 |
| | | | | US | 2021015858 | A1 | 21 January 2021 |
| | | | | US | 10973853 | B2 | 13 April 2021 |
| | | | | AU | 2019218516 | A1 | 21 November 2019 |
| | | | | AU | 2019218516 | B2 | 21 October 2021 |
| | | | | KR | 20210006530 | A | 18 January 2021 |
| CN | 111748580 | A | 09 October 2020 | None | | | |
| WO | 2019137548 | A1 | 18 July 2019 | AU | 2019207296 | A1 | 16 July 2020 |
| | | | | US | 2021095028 | A1 | 01 April 2021 |
| | | | | CA | 3088332 | A1 | 18 July 2019 |
| | | | | IL | 275737 | D0 | 31 August 2020 |
| | | | | CN | 111601826 | A | 28 August 2020 |
| | | | | KR | 20200109313 | A | 22 September 2020 |
| | | | | SG | 11202005595 Y | A | 29 July 2020 |
| | | | | JP | 2021510710 | A | 30 April 2021 |
| | | | | TW | 201932489 | A | 16 August 2019 |
| | | | | EP | 3740508 | A1 | 25 November 2020 |
| US | 2020255516 | A1 | 13 August 2020 | JP | 2020537509 | A | 24 December 2020 |
| | | | | EP | 3689909 | A1 | 05 August 2020 |
| | | | | TW | 201915026 | A | 16 April 2019 |
| | | | | CN | 111051347 | A | 21 April 2020 |
| | | | | WO | 2019062832 | A1 | 04 April 2019 |
| WO | 2016106302 | A1 | 30 June 2016 | AU | 2015369683 | A1 | 10 August 2017 |
| | | | | AU | 2015369683 | B2 | 10 December 2020 |
| | | | | PH | 12017501166 | A1 | 11 December 2017 |
| | | | | JP | 2018035138 | A | 08 March 2018 |
| | | | | JP | 6633032 | B2 | 22 January 2020 |
| | | | | US | 2021324072 | A1 | 21 October 2021 |
| | | | | CO | 2017006989 | A2 | 05 January 2018 |
| | | | | BR | 112017013385 | A2 | 06 February 2018 |
| | | | | PE | 20171244 | A1 | 24 August 2017 |
| | | | | IL | 285287 | D0 | 30 September 2021 |
| | | | | CL | 2017001660 | A1 | 23 March 2018 |
| | | | | TN | 2017000267 | A1 | 19 October 2018 |
| | | | | AR | 103268 | A1 | 26 April 2017 |
| | | | | MX | 2017007744 | A | 05 September 2017 |
| | | | | JP | 2017520512 | A | 27 July 2017 |
| | | | | JP | 6180663 | B2 | 16 August 2017 |

Form PCT/ISA/210 (patent family annex) (January 2015)

| INTERNATIONAL SEARCH REPORT<br>Information on patent family members | | International application No.<br>**PCT/CN2021/131743** | |
|---|---|---|---|
| Patent document<br>cited in search report | Publication date<br>(day/month/year) | Patent family member(s) | Publication date<br>(day/month/year) |
| | | TW 201629102 A | 16 August 2016 |
| | | TW I708786 B | 01 November 2020 |
| | | KR 20170099966 A | 01 September 2017 |
| | | EA 201791171 A1 | 30 November 2017 |
| | | EP 3237448 A1 | 01 November 2017 |
| | | AU 2021201231 A1 | 11 March 2021 |
| | | UY 36471 A | 30 June 2016 |
| | | SG 11201705063V A | 28 July 2017 |
| | | SG 10202006538 T A | 28 August 2020 |
| | | JP 2020062026 A | 23 April 2020 |
| | | IL 253013 D0 | 31 August 2017 |
| | | IL 253013 A | 31 August 2021 |
| | | US 2016176963 A1 | 23 June 2016 |
| | | US 10189902 B2 | 29 January 2019 |
| | | JP 2021177759 A | 18 November 2021 |
| | | ZA 201704981 B | 28 August 2019 |
| | | CN 107207594 A | 26 September 2017 |
| | | WO 2016106302 A9 | 22 June 2017 |
| | | CA 2971732 A1 | 30 June 2016 |
| | | CN 110256558 A | 20 September 2019 |
| | | MA 40662 A1 | 30 June 2020 |
| | | MA 40662 B1 | 31 December 2020 |
| | | US 2019112375 A1 | 18 April 2019 |
| | | US 11008390 B2 | 18 May 2021 |
| US 2018371083 A1 | 27 December 2018 | EP 3334757 A2 | 20 June 2018 |
| | | EP 3334757 A4 | 03 April 2019 |
| | | WO 2017030823 A2 | 23 February 2017 |
| | | WO 2017030823 A3 | 30 March 2017 |
| | | CN 108290936 A | 17 July 2018 |
| | | JP 2018527919 A | 27 September 2018 |
| | | JP 6976241 B2 | 08 December 2021 |
| | | AU 2016307845 A1 | 08 March 2018 |
| | | AU 2016307845 B2 | 15 October 2020 |
| | | US 10766957 B2 | 08 September 2020 |
| | | US 2021017276 A1 | 21 January 2021 |
| | | CA 2994555 A1 | 23 February 2017 |
| WO 2019129261 A1 | 04 July 2019 | JP 2021508676 A | 11 March 2021 |
| | | EP 3731868 A1 | 04 November 2020 |
| | | EP 3731868 A4 | 01 September 2021 |
| | | CN 111526888 A | 11 August 2020 |
| | | EA 202091587 A1 | 21 September 2020 |
| | | AU 2018393448 A1 | 09 July 2020 |
| | | US 2020331999 A1 | 22 October 2020 |
| | | CA 3086935 A1 | 04 July 2019 |
| | | TW 201930359 A | 01 August 2019 |
| | | SG 11202005213 W A | 29 July 2020 |
| | | IL 274934 D0 | 30 July 2020 |
| | | KR 20200105849 A | 09 September 2020 |
| | | BR 112020012647 A2 | 12 January 2021 |
| US 2018185480 A1 | 05 July 2018 | CA 2997130 A1 | 09 March 2017 |

Form PCT/ISA/210 (patent family annex) (January 2015)

| Patent document cited in search report | Publication date (day/month/year) | Patent family member(s) | | | Publication date (day/month/year) |
|---|---|---|---|---|---|
| | | JP | 2018532383 | A | 08 November 2018 |
| | | JP | 6861418 | B2 | 28 April 2021 |
| | | CN | 108137691 | A | 08 June 2018 |
| | | EP | 3344658 | A1 | 11 July 2018 |
| | | US | 10946095 | B2 | 16 March 2021 |
| | | WO | 2017037707 | A1 | 09 March 2017 |
| | | US | 2021220472 | A1 | 22 July 2021 |

**INTERNATIONAL SEARCH REPORT**
Information on patent family members

International application No.

**PCT/CN2021/131743**

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016106302 A1

- WO 2017053748 A2

**Non-patent literature cited in the description**

- **WATSON et al.** Molecular Biology of the Gene. The Benjamin/Cummings Pub.Co, 1987, 224